(19)

**Europäisches Patentamt**
**European Patent Office**
**Office européen des brevets**

(11)  **EP 1 425 028 B1**

(12)  **EUROPEAN PATENT SPECIFICATION**

(45) Date of publication and mention
of the grant of the patent:
**16.12.2009  Bulletin 2009/51**

(51) Int Cl.:
*A61K 38/17* *(2006.01)*     *A61K 39/395* *(2006.01)*
*A61K 48/00* *(2006.01)*     *A61P 31/00* *(2006.01)*

(21) Application number: **02736912.3**

(22) Date of filing: **16.05.2002**

(86) International application number:
**PCT/US2002/015556**

(87) International publication number:
**WO 2002/092008 (21.11.2002 Gazette 2002/47)**

(54) **USE OF IL-18 INHIBITORS FOR THE TREATEMENT OR PREVENTION OF SEPSIS**

VERWENDUNG VON IL-18-HEMMERN ZUR BEHANDLUNG ODER PRÄVENTION VON SEPSIS

UTILISATION D'INHIBITEURS DE IL-18 POUR LE TRAITEMENT OU LA PREVENTION DE LA SEPTICEMIE

(84) Designated Contracting States:
**AT BE CH CY DE DK ES FI FR GB GR IE IT LI LU MC NL PT SE TR**
Designated Extension States:
**AL LT LV MK RO SI**

(30) Priority: **16.05.2001  US 291463 P**

(43) Date of publication of application:
**09.06.2004  Bulletin 2004/24**

(73) Proprietor: **Yeda Research And Development Co. Ltd.**
**76100 Rehovot (IL)**

(72) Inventors:
• **DINARELLO, Charles, A.**
**Boulder, CO 80302 (US)**
• **FANTUZZI, Giamila**
**Denver, CO 80206 (US)**
• **KIM, Soo-Hyun**
**Aurora, CO 80013 (US)**
• **REZNIKOV, Leonid**
**Aurora, CO 80014 (US)**
• **RUBINSTEIN, Menachem**
**76329 Rehovot (IL)**

• **NOVICK, Daniela**
**Rehovot 76100 (IL)**
• **SCHWARTSBURD, Boris**
**76200 Rehovot (IL)**

(74) Representative: **Weiss, Wolfgang et al**
**Weickmann & Weickmann**
**Patentanwälte**
**Postfach 86 08 20**
**81635 München (DE)**

(56) References cited:
**EP-A- 0 974 600     WO-A-01/58956**
**WO-A-01/62285     WO-A-99/09063**
**US-A- 6 054 487     US-A- 6 083 981**

• **NETEA M G ET AL: "Neutralization of IL-18 reduces neutrophil tissue accumulation and protects mice against lethal Escherichia coli and Salmonella typhimurium endotoxemia." JOURNAL OF IMMUNOLOGY (BALTIMORE, MD. : 1950) 1 MAR 2000, vol. 164, no. 5, 1 March 2000 (2000-03-01), pages 2644-2649, XP002349802 ISSN: 0022-1767**

Description

FIELD OF THE INVENTION

The following application claims the benefit of U.S. Provisional Application No. 60/291,463 filed May 16, 2001,
[0002]    The present invention relates to the use of IL-18 inhibitors for treatment and /or prevention of sepsis related cardiac dysfunction.

BACKGROUND OF THE INVENTION

In 1989, a factor induced in the serum from infected mice with *mycobacterium bovis* and challenged with LPS was detected. This factor was capable of inducing interferon-γ (IFN-γ) in cultures of spleen cells of normal mice in the presence of interleukin-2 (IL-2) (Nakamura et al 1989). This factor functioned not as a direct inducer of IFN-γ but rather as a co-stimulant together with IL-2, anti-CD3 or mitogens. An attempt to further identify this activity from post-endotoxin treated mouse serum revealed an apparently homogeneous 50-55 kDa protein, which was associated with the above activity (Nakamura et al. 1993). Since other cytokines can act as co-stimulants for IFN-γ production, the failure of the neutralizing antibodies to IL-1, IL-4, IL-5, IL-6, or TNF to block the serum activity, suggested that this was a distinct factor. The same authors documented that the endotoxin-induced co-stimulant for IFN-γ production was present in extracts of livers from mice preconditioned with *P. acnes* (Okamura at al. 1995). According to this experimental model, the hepatic macrophage population (Kupffer cells) expands, and therefore a low dose of bacterial lipopolysaccharide (LPS) becomes lethal. Similar treatment is not lethal for non-pre-conditioned mice. The factor, named IFN-γ -inducing factor (IGIF) and later designated interleukin-18 (IL-18), was purified to homogeneity from these *P. acnes*-treated mouse livers and a partial amino acid sequence was obtained. Degenerate oligonucleotides derived from the amino acid sequences of purified IL-18 were used to clone a murine IL-18 cDNA (Okamura et al. 1995). The human cDNA sequence for IL-18 was reported in 1996 (Ushio et al. 1996).
Interleukin IL-18 (Tsutsui et al. 1996, Nakamura et al. 1993, Okamura et. al 1995, Ushio et al. 1996), shares structural features with the IL-1 family of proteins (Bazan et al. 1996), which have an all β-pleated sheet structure unlike most other cytokines, which exhibit a four-helix bundle structure. Similarly to IL-1β, IL-18 is synthesized as a biologically inactive precursor (proIL-18) and lacks a signal peptide (Ushio et al 1996). The IL-1β and IL-18 precursors are cleaved by caspase 1 (IL-1β-converting enzyme, or ICE), which cleaves the precursors after an aspartic acid residue in the P1 position. The resulting mature cytokines are released from the cell (Ghayur et al. 1997 and Gu et al. 1997), despite the lack of signal peptide.
IL-18 is known to act as a co-stimulant for cytokine production (IFN-γ, IL-2 and granulocyte-macrophage colony stimulating factor) by T helper type I (Th1) cells (Kohnoet al. 1997) and also a co-stimulant for FAS ligand-mediated cytotoxicity by murine natural killer cell clones (Tsutsui et al. 1996).
Interleukin-12 (IL-12) is an immunoregulatory cytokine produced by monocyte/macrophages and other antigen presenting cells. It is central to the orchestration of both innate and acquired cell-mediated immunoresponses (Trinchieri 1998). It consists of a heterodimer composed of the covalently linked products of two separate genes: a heavy chain (p40) and a light chain (p35). Production ofIL-12 is stimulated in response to certain bacteria, bacterial products, intracellular parasites and viruses. The following functions have been attributed to IL-12: 1) it is a potent inducer of IFN-γ from T and natural killer (NK) cells, 2) it is co-mitogenic for such cells, 3) it is critical for development of Th1 responses in most systems (thus leading secondarily, to increases in IFN-γ and TNF production, macrophage activation etc.), 4) it enhances cytotoxic T lymphocytes and NK cell cytotoxicity and 5) it is required for delayed-type hypersensitivity responses. IL-12 production in isolated murine splenic leukocytes treated with Staphylococcus aureus Cowan 1 strain, was shown to be suppressed by IFN-α or IFN-β (Karp et al. 2000).
Recently IL-12 has been reported to be involved in pathogenesis of organ-specific autoimmune inflammatory diseases such as multiple sclerosis (MS) (Karp et al. 2000) and inflammatory bowel disease (IBD) (Blumberg and Strober 2001 and Fiocchi 1999) and it is thus being regarded as a potential drug target for the treatment of these conditions.
IL-18 and IL-12 exhibit a marked synergism in induction of IFN-γ in T cells (Okamura et al. 1998). Investigations into the mechanism of this synergism have revealed that IL-12 up-regulates expression of both chains of the IL-18 receptor on cells producing IFN-γ (Kim et al. 2001). Although IL-12 and IL-18 activate both innate and acquired immunity, their excessive production by activated macrophages may induce multiple organ disorders including malfunction of the immune system (Seki et al. 2000).
Cytokine binding proteins (soluble cytokine receptors) are usually the extracellular ligand binding domains of their respective cell surface cytokine receptors. They are produced either by alternative splicing or by proteolytic cleavage of the cell surface receptor. These soluble receptors have been described in the past: for example. the soluble receptors for IL-6 and IFN-γ (Novick et al. 1989), TNF (Engelmann et al. 1989 and Engelmann et al. 1990). IL-1 and IL-4 (Maliszewski et al, 1990) and IFN-α/β (Novick et al. 1994, Novick et al. 1992). One cytokine-binding protein, named osteoprotegerin (OPG, also known as osteoclast inhibitory factor - OCIF), a member of the TNFR/Fas family, appears to be the first example of a soluble receptor that exists only as a secreted protein (Anderson et al. 1997, Simonet et al. 997, Yasuda et al. 1998).

An interleukin-18 binding protein (IL-18BP) was affinity purified, on an IL-18 column, from urine (Novick et al. 1999). IL-18BP abolishes IL-18 induction of IFN-γ, and IL-18 activation of NF-kB *in vitro.* In addition, IL-18-BP inhibits induction of IFN-γ in mice injected with LPS. The IL-18BP gene was localized to the human chromosome 11, and no exon coding for a transmembrane domain could be found in the 8.3 kb genomic sequence comprising the IL-18BP gene. Four isoforms of IL-18BP generated by alternative mRNA splicing have been found in humans so far. They were designated IL-18BP a, b, c, and d, all sharing the same N-terminus and differing in the C-terminus (Novick et al 1999). These isoforms vary in their ability to bind IL-18 (Kim et al. 2000). Of the four, human IL-18BP (hIL-18BP) isoforms a and c are known to have a neutralizing capacity for IL-18. The most abundant IL-18BP isoform, the spliced variant isoform a, exhibits a high affinity for IL-18 with a rapid on-rate and a slow off-rate, and a dissociation constant (Kd) of approximately 0.4 nM (Kim et al. 2000). IL-18BP is constitutively expressed in the spleen, and belongs to the immunoglobulin superfamily. The residues involved in the interaction of IL-18 with IL-18BP have been described through the use of computer modelling (Kim et al. 2000) and based on the interaction between the similar protein IL-1β with the IL-1R type I (Vigers et al. 1997). According to the model of IL-18 binding to the IL-18BP, the Glu residue at position 42 and Lys residue at position 89 of IL-18 have been proposed to bind to Lys-130 and Glu-114 in IL-18BP, respectively (Kim et al. 2000).

IL-18BP is constitutively present in many cells (Puren et al. 1999) and circulates in healthy humans (Urusbihara et al. 2000), representing a unique phenomenon in cytokine biology. Due to the high affinity of IL-1BP to IL-18 (Kd = 0.4 nM) as well as the high concentration of IL-18BP found in the circulation (20 fold molar excess over IL-18), it has been speculated that most, if not all of the IL-18 molecules in the circulation are bound to IL-18BP. Thus, the circulating IL-18BP that competes with cell surface receptors for IL-18 may act as a natural anti-inflammatory and an immunosuppressive molecule.

As mentioned, IL-18 induces IFN-γ which, in turn, was recently reported to induce IL-18BPa mRNA generation *in vitro* (Muhl et al 2000). Therefore. IL-18BPa could serve as a "shut off" signal, terminating the inflammatory response.

IL-18BP is significantly homologous to a family of proteins encoded by several Pox viruses (Novick et al.1999, Xiang and Moss 1999). Inhibition of IL-18 by this putative viral IL-18BP may attenuate the inflammatory antiviral Thl response.

The term systemic inflammatory response syndrome (SIRS) describes the familiar clinical syndrome of sepsis, independent of its cause. SIRS can result from trauma. pancreatitis, drug reactions, autoimmune disease, and other disorders: when it arises in response to infection, sepsis is said to be present (Nathens and Marshall 1996).

Septic shock is the most common cause of death in medical and surgical intensive - care units (Astiz and Rackow 1998). The terms sepsis, severe sepsis and septic shock are used to identify the continuum of the clinical response to infection. Patients with sepsis present evidences of infection and clinical manifestations of inflammation. Patients with severe sepsis develop hypoperfusion whith organ dysfunction. Septic shock is manifested by hypoperfusion and persistent hypotension. Mortality ranges from 16% in patients with sepsis to 40-60% in patients with septic shock. Bacterial infection is the most common cause of septic shock. The most frequent sites of infection are the lungs, abdomen, and urinary tract. General anti-inflammatory therapies such as the use of corticosteroids, failed to show improvement in survival from sepsis and septic shock. Three monoclonal antibodies specific to endotoxin have been tested in clinical trials and have failed to improve survival rates. Therapy with antagonists to tumor necrosis factor, interleukin 1, bradykinin, ibuprofen, and platelet activating-factor, did not show improvement on survival from septic shock (Astiz and Rackow 1998).

Sepsis is caused *inter alia* by Gram-positive bacteria e.g. *Staphylococcus epidermidis.* Lipoteichoic acids and peptidoglycans, which are the main cell wall components of the Staphylococcus species, are thought to be the inducers of cytokine release in this condition (Grupta et al. 1996 and Cleveland et al. 1996). However, other Gram-positive components are considered as stimulators of cytokine synthesis as well (Henderson et al. 1996). Production of IL-1, TNF-α and IFN-γ are thought to be the major contributors in the pathogenesis of septic shock (Dinarello 1996 and Okusawa et al. 1988). In addition, the chemokine IL-8 was shown to be induced by neutrophils in response to *S. epidermidis* (Hachicha et al. 1998). Important factors in the regulation of IL-1, TNF-α and IFN-γ induction by S epidermidis are IL-18, IL-12, IL-1β and TNF-α. IL-1β and TNF-α can be considered as a co-stimuli for IFN-γ production by T lymphocytes in a manner similar to IL-18. These two cytokines have a co-stimulatory activity on IFN-γ production in the context of IL-12 or bacterial stimulation (Skeen et al. 1995 and Tripp al al. 1993).

Recently it was reported by Nakamura et al. (2000) that concomitant administration of IL-18 and IL-12 to mice results in high toxicity similar to that found in endotoxin-induced septic shock.

The levels of IL-18 have been show to be elevated in sera from patients with sepsis (Endo et al. 2000) but this increase correlated with creatinine levels suggesting that the elevated levels of IL-18 may result from renal failure. In another study (Grobmyer et al. 2000), the levels of IL-18 in 9 subjects with sepsis during the first 96 hours following hospital admission were high and did not correlate with creatinine levels.

As is apparent from the above, IL-18 is a pleiotropic interleukin having both inflammatory enhancing and attenuating functions. On the one hand, it enhances production of proinflammatory cytokines like TNF-$\alpha$, therefore promoting inflammation. On the other hand, it induces the production of nitric oxide (NO), an inhibitor of caspase-1. thus blocking the maturation of IL-1$\beta$, and possibly attenuating inflammation. This dual role of IL-18, seriously questions the efficacy of IL-18 inhibitors in the treatment of inflammatory diseases. Furthermore, because of the involvement of a large variety of different cytokines and chemokines in the regulation of inflammation, it cannot always be expected to obtain a beneficial effect by blocking only one of the pathways in such a complicated interaction network.

Netea et al. (2000) reported that administration of anti-IL-18 polyclonal antibody protected mice against the deleterious effects of both LPS derived from *E. coli* or *S. typhimurium* species tested, supporting the concept that IL-18 has an important pathogenic role in lethal endotoxemia. However, the uncertainty regarding the efficiency of a potential treatment formulation based on administration of IL-18 inhibitors, is manifested in that IL-18 knock out mice are not less susceptible to sepsis as compared to wild type animals (Sakao et al. 1999) and in that recent reports suggest that the proinflammatory activity of IL-18 is essential to host defences against severe infections (Nakanishi et al. 2001 and Foss et al. 2001).

Thus, there exists a need to provide means to treat and/or prevent sepsis despite the above-mentioned considerations regarding the use of IL-18 inhibitors.

## SUMMARY OF THE INVENTION

The invention relates to the use of an inhibitor of IL-18 in the manufacture of a medicament for the treatment and/or prevention of sepsis related cardiac dysfunction, wherein the inhibitor is selected from antibodies against IL-18 and the IL-18 binding protein IL-18EPa and IL-18PBc.

The IL-18 binding protein used according to the invention is an IL-18BPa or EL-18BPc, a fused protein (e. g., Ig fused), functional derivative (e. g., PEG-conjugated), an active fraction or derivative thereof.

The antibodies used according to the invention may be anti IL-18 specific antibodies selected from chimeric, humanized and human antibodies.

In addition, the invention relates to the use of an inhibitor in the manufacture of a medicament further comprising a tumor necrosis factor $\alpha$ inhibitor, preferably soluble TNFRI or TNFRI1, and/or an IL-I$\beta$ inhibitor, preferably IL-I receptor antagonist for simultaneous, sequential or separate administration.

In one aspect the invention provides the use of an expression vector comprising the coding sequence of an inhibitor of IL-18 selected from antibodies against IL-18 and IL-18 binding proteins, IL-18BPa and IL-18BPc in the manufacture of a medicament for the treatment and/or prevention of sepsis related cardiac dysfunction, for example by gene therapy.

In another aspect, the invention provides the use of a vector for inducing and/or enhancing the endogenous production of an inhibitor of IL-18 selected from the IL-18 binding proteins IL-18BPa and IL-18BPc in a cell in the manufacture of a medicament for the treatment and/or prevention of sepsis related cardiac dysfunction.

In addition, the invention provides the use of a cell that has been genetically modified to produce an inhibitor of IL-18 selected from antibodies against IL-18 and the IL-18 binding proteins IL-18BPa and IL-18BPc in the manufacture of a medicament for the treatment and/or prevention of sepsis related cardiac dysfunction.

## BRIEF DESCRIPTION OF THE FIGURES

**Figure 1** shows the distribution of serum IL-18BPa in healthy individuals. Sera of male and female healthy individuals, ages 20-60, as assayed for the presence of IL-18BPa by a specific ELISA test (n=107).

**Figure 2 A** shows the average levels of IL-18 and IL-18BPa in sepsis patients and healthy subjects. Serum IL-18 and IL-18BPa were determined in the healthy individuals (see Figure 1) and in 198 samples from 42 sepsis patients immediately upon hospital admission and during hospitalisation.

**Figure 2 B** shows the distribution of the individual levels of IL-18 and IL-18BPa of the healthy subjects and patients described in Figure 2A. The mean serum levels of IL-18 and IL-18BPa in healthy subjects are indicated by a dashed vertical and horizontal line, respectively.

**Figure 3** shows a comparison between the total and free IL-18 in individual sepsis patients upon hospital admission. The level of free IL-18 (closed circles) in sera was calculated based on the concentration of total IL-18 (open circles) and IL-18BPa, taking into account a stoichiometry of 1:1 of IL-18 to IL-18BPa in a complex and a calculated Kd of 400pM. Each vertical line links total and free IL-18 in an individual serum sample.

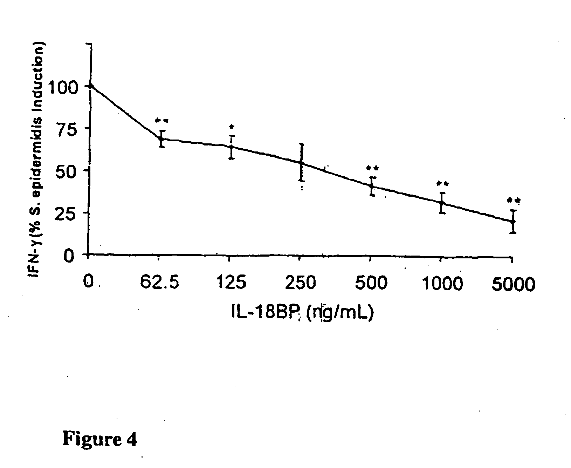

**Figure 4** shows the effect of IL-18BP on *S. epidermidis*-induced IFN-$\gamma$ production in whole blood. Whole blood was stimulated with *S. epidermidis* alone or S. *epidermidis* and recombinant IL-18BP at indicated concentrations. After 48 hours of incubation, the blood culture was lysed and IFN-$\gamma$ was measured. The results are expressed as percentage of *S. epidermidis* IFN-$\gamma$ induction. P<0.01 vs. *S. epidermidis* alone. The data represents the mean$\pm$ standard error of the mean (SEM) of six experiments. SEM represents the spread of the mean of a sample. SEM gives an idea of the accuracy of the mean. SEM = SD/(square root of sample size). The data was analysed with paired Student's test.

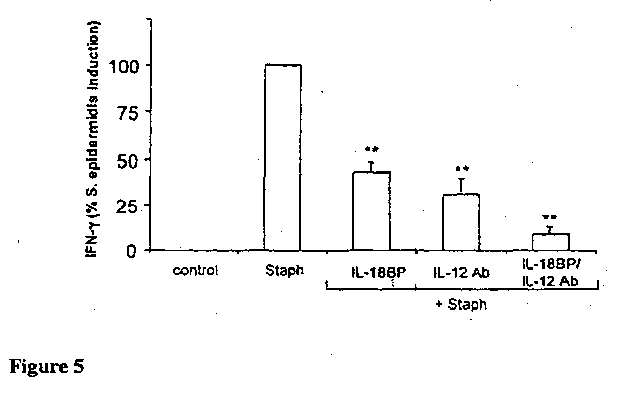

**Figure 5** shows the inhibitory effect exerted by the double activity of IL-18BPa and anti IL-12 monoclonal antibodies on the production of IFN-γ by whole blood treated with S. *epidermidis.* Whole blood was stimulated with *S. epidermidis* in the presence or in the absence or of IL-18BPa (125 ng/ml), IL-12. Mab (2.5μg/ml) and both. After 48 hours of incubation, the blood culture was lysed and IFN-γ was measured. The data is expressed as percent of IFN-γ induction. P<0.01 vs. *S. epidermidis* alone. The data represents the mean± standard error of the mean (SEM) of six experiments. SEM represents the spread of the mean of a sample. SEM gives an idea of the accuracy of the mean. SEM = SD/(square root of sample size). The data was analysed with paired Student's test.

**Figure 6** shows the quantitation of IL-18BPa as reflected in an ELISA standard curve. Recombinant human IL-18BPa was serially diluted and subjected to ELISA as described in example 3. The data shows the mean absorbance ± SE (standard error) of 10 experiments.

**Figure 7** shows the inhibitory effect of IL-18 on the quantitation of IL-18BPa by ELISA assay. The percent of inhibition of the signal is shown.

**Figure 8** shows the immunological cross reactivity of human IL-18BP isoforms. The IL-18P ELISA was performed with all isoforms (a-d) of IL-18BP. Stocks (3.2 μg/ml) of the IL-18BP isoforms were serially diluted and tested in IL-18BPa ELISA.

**Figure 9** shows myocardial tissue content of IL-18 following LPS administration. Mice were injected with E.coli LPS (0.5 mg/Kg, intraperitonially). Hearts were homogenized at the indicated times at the x taxis. ELISA was carried out to determine myocardial IL-18 content. (n=4-5 per group).

**Figure 10** shows the effect of anti IL-18 antibody on LPS-induced myocardial dysfunction. Mice were injected with either vehicle (intraperitoneally injected saline n=8) or E.coli. LPS (0.5 mg/Kg injected intraperitoneally, n=8) and the left ventricular developed pressure (LVDP) was determined at 6 hours by isolated heart perfusion. In separate experiments, mice were pretreated with either normal rabbit serum (NRS, n=5) or anti-IL-18 antibody (Anti IL-18, n=8) 30 minutes prior to LPS.

## DETAILED DESCRIPTION OF THE INVENTION

The present invention relates to the administration of an IL-18 inhibitor selected from antibodies against IL-18 and the IL-18 binding proteins IL-18BPa and IL-18BPc to prevent or treat sepsis related cardiac dysfunction and is based on the findings described in the examples. It was found in according with the present invention that the levels of effective (free) circulating IL-18 are significantly elevated in serum of sepsis patients in comparison to healthy individuals, and that the inhibition of IL-18 causes a decrease in IFN-γ induction by the Gram-positive bacterium *Staphylococcus epidermidis.*

In addition the prevention treatment of sepsis related cardiac dysfunction can be approached by administration of an IL-18 inhibitor in combination of other cytokine inhibitors potentially able to increase its effect.

Sepsis according to the present invention comprises SIRS, severe sepsis, septic shock, endotoxic shock etc. and may be caused by Gram-positive or Gram-negative bacteria

Inhibitors of IL-18 action can be IL-18 antagonist for example IL-18BPa and IL-18BPc. Antagonists can either bind to or sequester the IL-18 molecule itself with sufficient affinity and specificity to partially or substantially neutralize the IL-18 or IL-18 active site(s) responsible for IL-18 binding to its ligands (e. g., to its receptors).

Inhibitors of IL-18 action may be also IL-18 antibodies such as monoclonal antibodies.

The term "IL-18 binding proteins" is used herein synonymously with "IL18-BP". It comprises IL-18 binding proteins IL-18BPa and IL-18BPc. In particular, human isoforms a and c of IL-18BP are useful in accordance with the presence invention. The proteins useful according to the present invention may be glycosylated or non-glycosylated, they may be derived from natural sources, such as urine, or they may preferably be produced recombinantly. Recombinant expression may be carried out in prokaryotic expression systems like *E. coli,* or in eukaryotic, and preferably in mammalian, expression systems.

The term "fused protein" refers to a polypeptide comprising an IL-18BPa or IL-18BPc fused with another protein, which, e. g., has an extended residence time in body fluids. An IL-18BP may thus be fused to another protein, polypeptide or the like, e.g. an immunoglobulin or a fragment thereof.

"Functional derivatives" as used herein cover derivatives of IL-18BPs and their fused proteins, which may be prepared from the functional groups which occur as side chains on the residues or the N- or C-terminal groups, by means known in the art, and are included in the invention as long as they remain pharmaceutically acceptable, i.e., they do not destroy the activity of the protein which is substantially similar to the activity of IL-18BP and do not confer toxic properties on compositions containing it.

These derivatives may, for example, include polyethylene glycol side-chains (PEG-conjugated), which may mask antigenic sites and extend the residence of an IL-18BP or a viral IL-18BP in body fluids. Other derivatives include aliphatic esters of the carboxyl groups, amides of the carboxyl groups by reaction with ammonia or with primary or secondary amines, N-acyl derivatives of free amino groups of the amino acid residues formed with acyl moieties (e.g., alkanoyl or carbocyclic aroyl groups) or O-acyl derivatives of free hydroxyl groups (for example that of seryl or threonyl residues) formed with acyl moieties.

As "active fractions" of an IL-18BP and fused proteins, the present invention covers any fragment or precursors of the polypeptide chain of the protein molecule alone or together with associated molecules or residues linked thereto, e.g., sugar or phosphate residues, or aggregates of the protein molecule or the sugar residues by themselves, provided said fraction has substantially similar activity to IL-18BP.

Functional derivatives of IL-18BP may be conjugated to polymers in order to improve the properties of the protein, such as the stability, half-life, bioavailability, tolerance by the human body, or immunogenicity. To achieve this goal, IL18-BP may be linked e.g., to Polyethlyenglycol (PEG). PEG-conjugated may be carried out by known methods described in WO 92/13095, for example.

Therefore, in a preferred embodiment of the present invention. IL-18BP is PEG-conjugated.

In a further preferred embodiment of the invention, the inhibitor of IL-18 is a fused protein comprising all or part of an IL-18 binding protein, which is fused to all or part of an immunoglobulin. The person skilled in the art will understand that the resulting fusion protein retains the biological activity of IL-18BP, in particular the binding to IL-18. The fusion may be direct, or via a short linker peptide which can be as short as 1 to 3 amino acid residues in length or longer, for example. 13 amino acid residues in length. Said linker may be a tripeptide of the sequence E-F-M (Glu-Phe-Met), for example, or a 13-amino acid linker sequence comprising Glu-Phe-Gly-Ala-Gly-Leu-Val-Leu-Gly-Gly-Gln-Phe-Met introduced between the IL-18BP sequence and the immunoglobulin sequence. The resulting fusion protein has improved properties, such as an extended residence time in body fluids (half-life), increased specific activity, increased expression level, or the purification of the fusion protein is facilitated.

IL-18BP may be fused to the constant region of an Ig molecule. Preferably, it is fused to heavy chain regions, like the CH2 and CH3 domains of human IgG1, for example. The generation of specific fusion proteins comprising IL-18BP and a portion of an immunoglobulin are described in Example 11 of WP99/09063, for example. Other isoforms of Ig molecules are also suitable for the generation of fusion proteins according to the present invention, such as isoforms $IgG_2$ or $IgG_4$, or other Ig classes, like IgM or IgA, for example. Fusion proteins may be monomeric or multimeric, hetero- or homomultimeric.

The inhibitor of IL-18 can be an anti-IL-18 specific antibody. Anti-IL-18 antibodies may be polyclonal or monoclonal, chimeric, humanized, or even fully human. Recombinant antibodies and fragments thereof are characterized by high affinity binding to IL-18 *in vivo* and low toxicity. The antibodies which can be used in the invention are characterized by their ability to treat patients for a period sufficient to have good to excellent regression or alleviation of the pathogenic condition or any symptom or group of symptoms related to a pathogenic condition, and a low toxicity.

IL- 18 has been shown to increase in the course of cardiac dysfunction caused by sepsis Co-administration of LPS together with an IL-18 inhibitors such as a neutralizing anti-IL-18 polyclonal antibody, protects from LPS-induced myocardial dysfunction.

Neutralizing antibodies are readily raised in animals such as rabbits, goat or mice by immunization with IL-18. Immunized mice are particularly useful for providing sources of B cells for the manufacture of hybridomas, which in turn are cultured to produce large quantities of anti-IL-18 monoclonal antibodies.

Chimeric antibodies are immunoglobulin molecules characterized by two or more segments or portions derived from different animal species. Generally, the variable region of the chimeric antibody is derived from a non-human mammalian antibody, such as murine monoclonal antibody, and the immunoglobulin constant region is derived from a human immunoglobulin molecule. Preferably, both regions and the combination have low immunogenicity as routinely determined (Elliott et al., 1994) Humanized antibodies are immunoglobulin molecules created by genetic engineering techniques in which the murine constant regions are replace with human counterparts while retaining the murine antigen binding regions. The resulting mouse-human chimeric antibody preferably have reduced immunogenicity and improved pharmacokinetics in humans (Knight et al., 1993).

Thus, in a further preferred embodiment, IL-18 antibody is a humanized IL-18 antibody. Preferred examples of humanized anti-IL-18 antibodies are described in the European Patent Application EP 0 974 600, for example.

In yet a further preferred embodiment, the IL-18 antibody is fully human. The technology for producing human antibodies is described in detail e.g., in WO00/76310, WO99/53049, US 6,162,963 or AU5336100. Fully human antibodies are preferably recombinant antibodies, produced in transgenic animals, e.g., xenomice, comprising all or parts of functional human Ig loci.

The polypeptide inhibitors can be produced in prokaryotic or eukaryotic recombinant systems or can be encoded in vectors designed for gene targeting.

Other cytokine inhibitors can be used together with inhibitors of IL-18 in the treatment of sepsis related cardiac dysfunction. An inhibitor of IL-18 may be used in combination with an IL-12 inhibitor.

In addition. an inhibitor of IL-18 may be used in combination with inhibitors of other cytokines, known to play an important role in septic shock e.g., IL-I. TNF. IL.8 etc Dinarello 1996 and-Okusawa et al. 1988. An example for an inhibitor of IL- is IL I-receptor antagonist, and for TNF the soluble portion of receptors TNFR1 and TNFRI2.

The term "inhibition of cytokines" within the context of this invention refers to any molecule modulating the referred cytokine production (e.g.. ICE inhibitors in the case of IL-1) and/or action in such a way that its production and/or action is attenuated, reduced, or partially, substantially or completely prevented or blocked.

An inhibitor of production can be any molecule negatively affecting the synthesis, processing or maturation of said cytokine. The inhibitors, considered according to the invention can be, for example, suppressors of gene expression of the cytokine, antisense mRNAs reducing or preventing the transcription of the cytokine mRNA or ribozymes leading to degradation of the mRNA, proteins impairing correct folding, or partially or substantially preventing secretion of said cytokine, proteases degrading the cytokine and the like.

Cytokine antagonists exert their activity in several ways. Antagonists can bind to or sequester the cytokine molecule itself with sufficient affinity and specificity to partially or substantially neutralize the cytokine epitope or epitopes responsible for the cytokine receptor binding (hereinafter termed "sequestering antagonists"). A sequestering antagonist may be, for example, an antibody directed against the cytokine, a truncated form of the cytokine receptor (e.g., soluble receptor TNFRI and TNFRII in the case of TNF), comprising the extracellular domains of the receptor or functional portions thereof etc. Alternatively, cytokine antagonists can inhibit the cytokine-signaling pathway activated by the cell surface receptor after cyrtokine binding (hereinafter termed "signaling antagonists"). An antagonist can be also a molecule that binds the receptor but is not able to trigger the signaling pathway activated upon binding of the cytokine to said receptor (e.g.. IL1-Ra Dinarello 1996). All groups of antagonists are useful, either alone or together, in combination with an IL-18 inhibitor, in the therapy of sepsis.

The IL-18 inhibitor can be used simultaneously, sequentially or separately with the above described cytokine inhibitors.

The invention further relates to the use of an expression vector comprising the coding sequence of an inhibitor of IL-18 selected from antibodies against IL-18 and the IL-18 binding proteins IL-18BPa and IL-18BPc in the preparation of a medicament for the prevention and/or treatment of sepsis related cardiac dysfunction. A gene therapeutical approach is thus used for treating and/or preventing the disease. Advantageously, the expression of the IL-18 inhibitor will then be in situ, thus efficiently blocking IL-18 directly in the tissue(s) or cells affected by the disease.

The use of a vector for inducing and/or enhancing the endogenous production of an inhibitor of IL-18 selected from IL-18 binding proteins IL-18BPa and IL-18BPc in a cell normally silent for expression of an IL-18 inhibitor, or which expresses amounts of the inhibitor which are not sufficient, are also contemplated according to the invention. The vector may comprise regulatory sequences functional in the cells desired to express the inhibitor or IL-18. Such regulatory sequences may be promoters or enhancers, for example. The regulatory sequence may then be introduced into the right locus of the genome by homologous recombination, thus operably linking the regulatory sequence with the gene, the expression of which is required to be induced or enhanced. The technology is usually referred to as "endogenous gene activation" (EGA), and it is described, e. g., in WO 91/09955.

The invention comprise also the administration of genetically modifid cells, able to produce an inhibitor for the treatment or prevention of sepsis related cardiac dysfunction.

The definition of pharmaceutically acceptable" is meant to encompass any carrier, which does not interfere with effectiveness of the biological activity of the active ingredient and that is not toxic to the host to which it is administered. For example, for parenteral (e.g., intravenous, subcutaneous, intramuscular) administration, the active protein(s) may be formulated in a unit dosage form for injection in vehicles such as saline, dextrose solution, serum albumin and Ringer's solution.

The active ingredients of the pharmaceutical composition according to the invention can be administered to an individual in a variety of ways. The routes of administration include intradermal, transdermal (e.g., in slow release formulations), intramuscular, intraperitoneal, intravenous, subcutaneous, oral, epidural, topical, and intranasal routes. Any other therapeutical efficacious route of administration can be used, for example absorption through epithelial or endothelial tissues or by gene therapy wherein a DNA molecule encoding the active agent is administered to the patient (e.g. via a vector), which causes the active agent to be expressed and secreted in vivo. In addition, the protein(s) according to the invention can be administered together with other components of biologically active agents such as pharmaceutically acceptable surfactants, excipients, carriers, diluents and vehicles.

For parenteral (e.g.. intravenous, subcutaneous, intramuscular) administration, the active protein(s) can be formulated as a solution, suspension, emulsion or lyophilised powder in association with a pharmaceutically acceptable parenteral vehicle (e.g.. water, saline, dextrose solution) and additives that maintain isotonicity (e.g., mannitol) or chemical stability (e.g., preservatives and buffers). The formulation is sterilized by commonly used techniques.

The bioavailability of the active protein(s) according to the invention can also be ameliorated by using conjugation procedures which increase the half-life of the molecule in the human body, for example linking the molecule to polyethylenglycol, as described in the PCT Patent Application WO 92/13095.

The therapeutically effective amounts of the active protein(s) will be a function of many variables, including the type of antagonist, the affinity of the antagonist for IL-18, any residual cytotoxic activity exhibited by the antagonists, the route of administration, the clinical condition of the patient (including the desirability of maintaining a non-toxic level of endogenous IL-18 activity).

A "therapeutically effective amount" is such that when administered, the IL-18 inhibitor results in inhibition of the biological activity of IL-18. The dosage administered, as single or multiple doses, to an individual will vary depending upon a variety of factors, including IL-18 inhibitor pharmacokinetic properties, the route of administration, patient conditions and characteristics (sex, age, body weight, health, size), extent of symptoms, concurrent treatments, frequency of treatment and the effect desired. Adjustment and manipulation of established dosage ranges are well within the ability of those skilled in the art, as well as in vitro and in vivo methods of determining the inhibition of IL-18 in an individual.

According to the invention, the IL-18 inhibitor can be administered prophylactically or therapeutically to an individual in need prior to, simultaneously or sequentially with other therapeutic regimens or agents (e.g., multiple drug regimens), in a therapeutically effective amount, in particular with an IL-12 inhibitor and/or IL-1 inhibitor, interferon and TNF antagonist. Active agents that are administered simultaneously with other therapeutic agents can be administered in the same or different compositions.

Further disclosed is a method for the preparation of a pharmaceutical composition comprising admixing an effective amount of an IL-18 inhibitor and/or and IL-12 inhibitor and/or IL-1 inhibitor, interferon and/or a TNF antagonist with a pharmaceutically acceptable carrier.

Having now described the invention it will be more readily understood by reference to the following examples that are provided by way of illustration.

## EXAMPLE

### Example 1:

**Levels of serum IL-18BPa and IL-18 in healthy individuals and sepsis patients.**

Measurement of specific circulating cytokines and their natural inhibitors in health and disease provides information about their involvement in progression and severity of a disease. As mentioned in the background section, one of the key mediators of sepsis is IFN-$\gamma$. Since IL-18 is a coinducer of IFN-$\gamma$, the level of IL-18 and its natural inhibitor, IL-18BP splice variant a, were monitored in sepsis patients and compared to the levels found in healthy subjects by using specific ELISAs (example 3).

Levels of IL-18 and IL-18BPa in healthy subjects.

The mean level of IL-18 in 107 healthy donors was $64 \pm 17$ pg/ml as measured by the ECL assay (Pomerantz et al. 2001). The ECL assay was tested for interference by related proteins. It was found that it was no affected by the presence of mature IL-1$\beta$ or proIL 1$\beta$, whereas pro-IL-18 was cross-reactive. Therefore, as much as 20% of the detected mature IL-18 in human serum samples in the present study may be pro-IL-18. The ECL assay of IL-18 was not affected by IL 18BPa at a concentration $\leq$ 160 ng/ml.

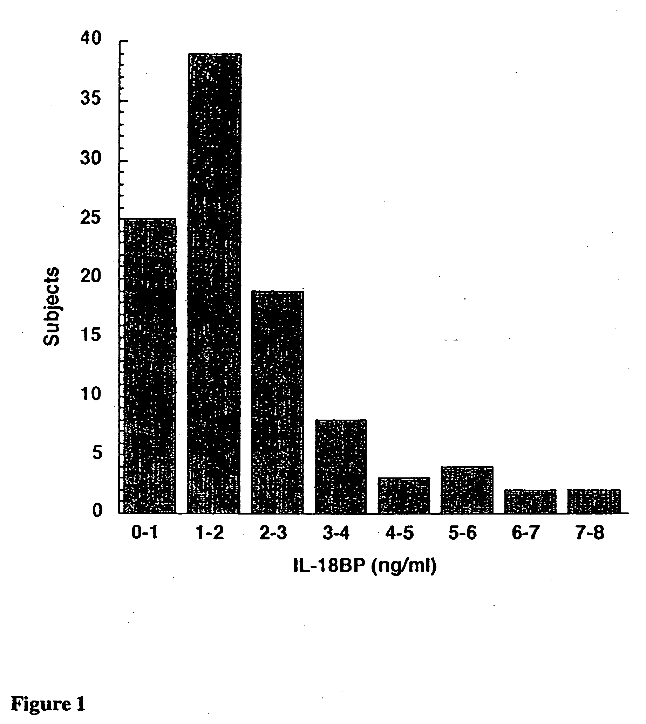

The levels of IL-18BPa were tested in the serum from the 107 healthy individuals by ELISA (example 3). The levels of IL-18BPa ranged from 0.5 ng/ml to as high as 7 ng/ml, with an average of $2.15 \pm 0.15$ ng/ml (Figure 1).

Because both IL-18 and IL-18BPa are concomitantly present in the serum, some of the IL=18 may be present in a complex with IL-18BPa. The level of free IL-18 was calculated based on the average level of total IL-18 (2.15 ng/ml). Free IL-18 was determined according to the law of mass action. The calculation was based on the following parameters: the concentrations of total IL-18 as determined by the ECL assay; the concentration of total IL-18BPa as determined by the ELISA; a 1:1 stoichiometry in the complex of IL-18BPa and IL-18 and a dissociation constant (Kd) of 0.4 nM (Novick et al. 1999 and Kim et al. 2000). In an equilibrium system L+R $\rightleftarrows$ LR where L represents IL-18 and R represents IL-18BP the following equations are applicable:

$$Kd=[LR]/[L_{free}][R_{free}]$$

$$L_{free}=L_{total}-LR$$

$$R_{free}=R_{total}-LR$$

By substituting: $L_{total} = 64 \pm 17$ pg/ml (mean level). $R_{total} = 2.15 \pm 0.15$ ng/ml (average) and Kd=0.4 nM, it was found that in healthy subjects about 51.2 pg/ml IL-18 (about 80 % from total) is in its free form.

Levels of IL-18 and IL-18BPa in sepsis patients.

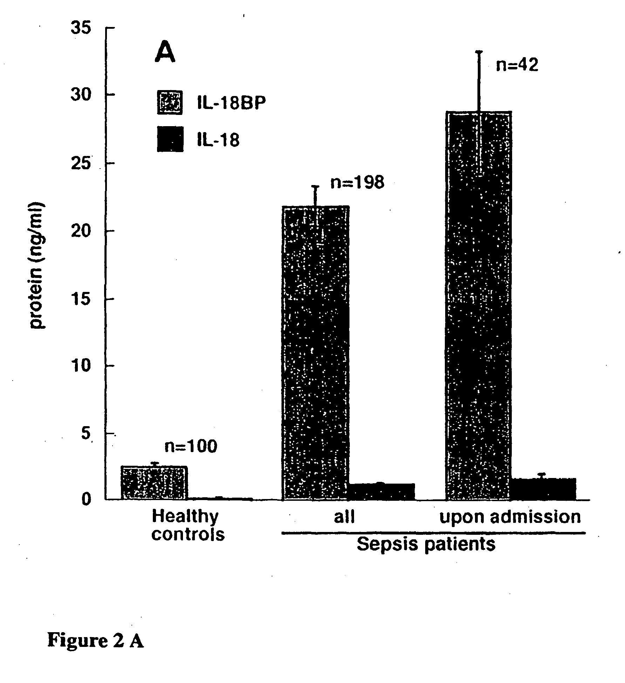

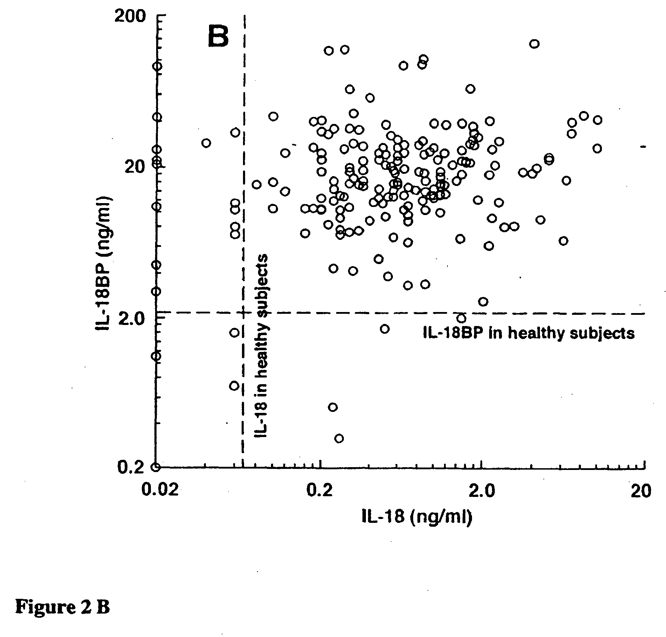

The levels of IL-18 and of IL-18BPa were tested in 192 sera samples from 42 septic patients immediately upon admission and during hospitalisation. The levels of both IL-18 and IL-18BPa were significantly more elevated in sepsis patients in comparison with the healthy subjects (Figure 2 A), and a broad distribution of the values was observed (Figure 2 B). Moreover, these levels were even higher in these patients at the day of admission, showing a 22 fold increase in the level of IL-18 compared with healthy individuals (1.5±0.4 ng/ml versus 0.064 ± 0.17 ng/ml) and a 13 fold increase in the level of IL-18BPa (28.6±4.5 versus 2.15±0.15 ng/ml, Figure 2A). No statistically significant correlation between creatinine levels and either IL-18 or IL-18BPa concentrations in these sera could be observed (assessed by APACHE II score Knaus et al. 1993), suggesting that elevated IL-18 and IL-18BPa levels in these patients were not due to renal failure.

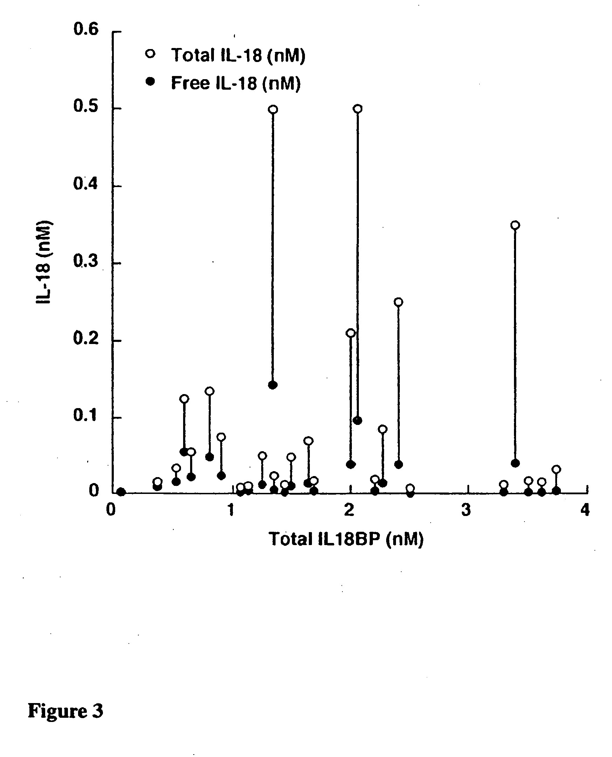

Because serum IL-18 and IL-18BPa levels in sepsis patients varied considerably (Figure 2B) the levels of free serum IL-18 in individual samples were calculated. The calculations were done as previously described, using the same three equations and substitution of the Kd, $L_{total}$, Rtotal values found experimentally. The calculations show that IL-18BPa reduced the level of free IL-18 in most patients (Figure 3). Notably, this effect was particularly strong when total IL-18 was very high. For example, in two patients, serum IL-18 reached 0.5 nM (10ng/ml), a 150-fold increase over healthy individuals. However, considering binding to the circulating IL-18BPa (27 ng/ml and 41.2 ng/ml), the level of free IL-18 in these two patients dropped by 78% and 84%, respectively (Figure 3), Therefore, most of the serum IL-18 is blocked by the circulating IL-18BPa. Yet, according to these calculations, the remaining free IL-18 is still higher than that found in healthy individuals. Therefore, administration of exogenous IL-18BPa to septic patients is expected to further lower the free circulating IL-18 level, causing alleviation of the disease outcome.

**Example 2. Effect of IL-18BPa on *S. epidermidis*-induced IFN-γ production in cells present in whole blood samples from healthy subjects.**

As mentioned in the background section the Gram-positive bacteria Staphylococcus *epidermidis* is known to cause sepsis. Induction of cytokines e.g. IL-1, IFN-γ and TNF-α are key mediators for this pathology. Since IL-18 is a co-inducer of IFN-γ, the effect of its inhibition on *Staphylococcus epidermidis* IFN-γ induction, was tested. The IL-18BPa, used as the IL-18 inhibitor, was a recombinant histidine tagged version produced in CHO cells.

0.5 ml of blood was mixed in 5 ml 12x75 mm round-bottom polypropylene tubes (Falcon, Becton Dickinson Labware, Franklin Lakes, NL) with 0.5 ml of RPMI growth medium (Cellgro Mediatech, Hendon. VA supplemented with 10 mM L-glutamine, 100U/ml penicillin 100μg/ml streptomycin and 10% FBS [Gibco BRL, Grand Island, NY]) containing *S. epidermidis* (from ATCC) with or without IL-18BP. The samples were incubated at 37°C (5% $CO_2$) for 48 hours followed by treatment with triton X-100 (Bio-Rad Laboratories, Richmond, CA) at 0.5% final concentration to lyse the cells present in the whole blood sample. The tubes containing the samples were inverted several times until the blood sample showed a clear appearance. IFN-γ concentration in the lysed samples of blood, which represents , both intracellular and secreted cytokine, was measured by electrocherniluminescence (ECL) as described by Pomerantz et al. (2001).

For this study whole blood from healthy, non-smoking volunteers was used. The blood was taken by venipuncture and kept in heparinized tubes. *S epidermidis* was propagated in brain-heart infusion broth for 24 hours, washed in pyrogen free saline and boiled as described by Aiura et al. (1993). The concentration of heat-killed bacteria was adjusted to 10 bacterial cells per white blood cell (WBC)

[0091] [00110] The results in Figure 4 show that IL-18BPa is able to inhibit the *S. epidermidis* induction of IFN-γ.

Since IFN-γ is known to be co-stimutated by IL-18 and IL-12, the effect of the combination of IL-18BPa and anti IL-12 specific antibodies (anti human IL-12 Mab.11.5.14, Preprotech, Rocky Hill, NJ) on IFN-γ induction directed by *S. epidermidis,* was tested. This induction was tested in the presence of 125 ng/ml IL-18BPa (histidine tagged IL-18BPa produced in COS cells and purified over a talon column as described by Novick et al. 1999) and 2.5 μg/ml anti human IL-12 specific antibody. The results shown in Figure 5 suggest that anti IL-12 specific antibodies potentiate the inhibitory effect of IL-18BPa on the IFN-γ induction by *S. epidermidis.*

**Example 3 development of an IL-18BP specific ELISA test.**

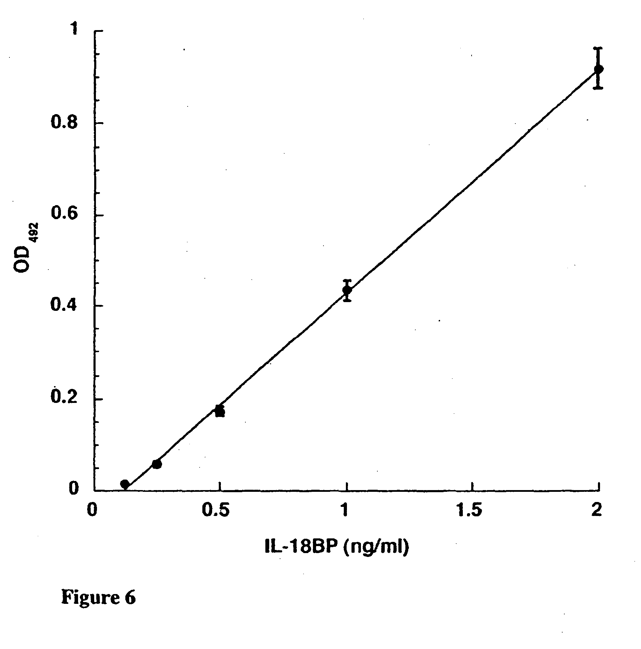

ELISA comprised two anti IL-18BPa antibodies: murine Mab, 582.10, a subclone of Mab 582 described in Example 4, as the capture antibody and rabbit polyclonal antibody for detection (Example 4). Microtiter 96-well ELISA plates (Maxisorb; Nunc A/S, Roskilde, Denmark) were coated with anti IL-18BPa MAb No. 582.10 (a subclone of MAb 582. 4 μg/ml in PBS) overnight at 4°C. The plates were washed with PBS containing 0.05% Tween 20 (washing solution) and blocked (2 h, 37°C) with BSA stock solution (KPL, Geithersburg, MD) diluted 1:10 in water. BSA stock solution was diluted 1:15 in water (diluent) for the dilution of all tested samples and the detecting antibody. Sera samples were diluted at least 1:5 in the diluent and 100 µl aliquots were added to the wells. Highly pure rIL-18BPa (prepared in CHO and purified by immunoaffinity using protein G-purified Mab N 430 specific for IL-18BP shown in table I example 4) was diluted by 7 serial two-fold dilutions (4 to 0.062 ng/ml) and added to each ELISA plate for the generation of a standard curve. The plates were incubated for 2 hrs at 37°C and washed 3 times with the w-ashing solution. Rabbit anti IL-18BPa serum (1:5000 in diluent, 100 µl/well) was added and the plates were further incubated for 2 hrs at 37°C. The plates were washed 3 times, a conjugate of goat-anti-rabbit horseradish peroxidase (HRP; Jackson ImmunoResearch Labs, 1:10,000 in PBS, 100 pl/well) was added and the plates were incubated for 1 h at 37°C. The plates were washed 3 times and developed by the addition of. OPD Peroxidase substrate (o-phenylenediamine dihydrochloride tablets, Sigma) for 30 min at room temperature. The reaction was stopped by 3N HCl (100 µl) and the absorbance at 492 nm was determined by an ELISA reader. The OD was plotted as a function of IL-18BPa concentration and linearity was observed in a range of 0.12-2.00 ng/ml IL-18BPa (Figure 6).

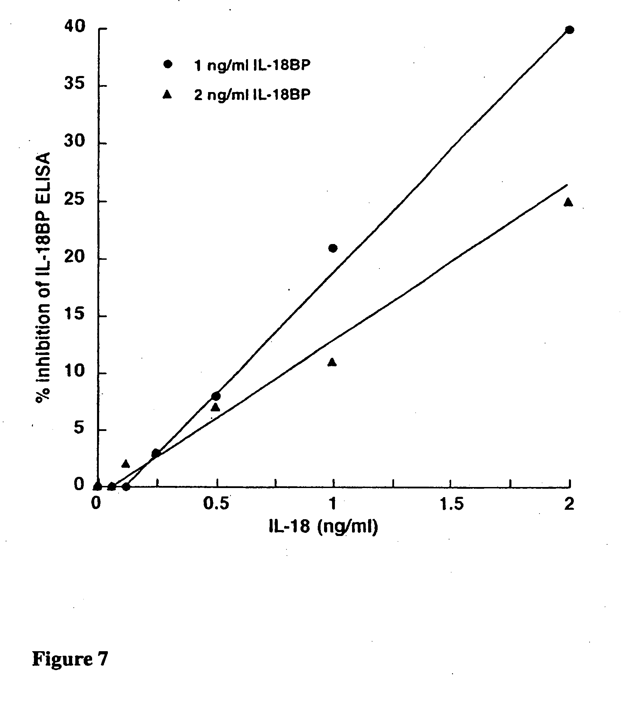

The IL-18BPa standard curve in the absence of serum or in the presence of up to 20% human serum remained the same. Since IL-18BPa binds IL-18 with a very high affinity, it was necessary to determine whether ELISA distinguishes between free and bound IL-18BPa. It was found that IL-18 interferes with the IL-18 ELISA, however, the interference is insignificant in serum samples. In 90% of sepsis patients, serum IL-18 levels were under 4 ng/ml (see below). Such sera samples have elevated IL-18BPa, requiring a 5 to 20-fold dilution prior to measuring IL-18BPa. At such dilutions, IL-18 interference with the IL-18BPa ELISA is less than 10% (Figure 7). Other related cytokines, including proIL-18, used at a 10-fold molar excess over IL-1 8BPa, as well as a 200-fold excess of mature IL-1β do not interfere with the assay.

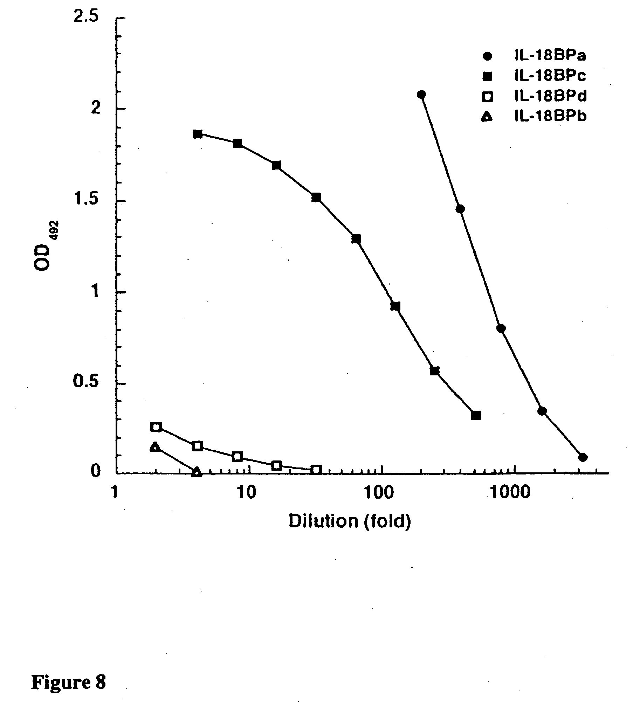

The most abundant isoform of human IL-18BP is IL-18BPa, whereas isoforms b, c and d are minor splice variants (Novick et al. 1999 and Kim et al. 2000). IL-18BPa exhibits the highest affinity for IL-18 (Kim et al 2000). The affinity of IL-1 8BPc for IL-18 is 10 fold lower, whereas isoforms b -and-d do not bind or neutralize IL-18. It was therefore important to determine the cross-reactivity of IL-18BPa in ELISA with the other isoforms of IL-18BP. As shown in Figure 8, human IL-18BPc generated a 10-fold lower signal on a weight basis compared with huIL-I8BPa, whereas human isoforms b and d generated an insignificant signal in the ELISA.

## Example 4: Generation of anti IL-18BP specific antibodies.

A rabbit was injected with rIL-I8BPa-His6 for the generation of polyclonal antibodies.

For the production of monoclonal antibodies, female Balb/C mice were injected 5 times with 10 µg of recombinant histidine tagged IL-18BPa (rIL-18BPa-His6). The mouse exhibiting the highest titer as determined by an inverted radioimmunoassay (IRIA example 5) or solid phase RIA (sRIA, example 5) was given a final boost intraperitoneally 4 and 3 days before fusion. Lymphocytes were prepared from spleen and fusion to NSO/1 myeloma cells was performed. Hybridomas that were found to produce antibodies to IL-18BPa were subcloned by limiting dilution. The clones generated were assayed by IRIA (Example 5). Representative hybridomas are shown in Table 1.

### Table 1 representative hybridomas producing anti IL-18BPa

| MAb No. | IRIA$_1$ (cpm) | sRIA$_2$ (cpm) |
|---------|----------------|----------------|
| 148 | 23912 | 7941 |
| 297 | 1652 | 6483 |
| 369 | 316 | 12762 |
| 430 | 6887 | 3254 |
| 433 | 1009 | 15300 |
| 460 | 3199 | 4326 |
| 485 | 400 | 13010 |
| 582 | 15000 | 17897 |
| 601 | 1046 | 1928 |

$_1$ Plates coated with goat anti mouse antibodies and hybridomas detected with 125I IL 18BPa.

$_2$ Plates coated with urinary IL 18BP and hybridomas detected with 1251 goat anti mouse antibodies.

Hybridomas secreting antibodies directed against the histidine tag were discarded. Antibodies were further characterized by sRIA for their ability to recognize the naturally occurring IL-18BP (purified from urine). Binding characteristics of several positive hybridomas are shown in Table 1. Hybridomas No. 148, 430, 460 and 582 were positive with both, recombinant and urinary IL-18BP. Antibodies suitable for Western blotting, immunoprecipitation, immunoaffinity purification and for development of a specific ELISA were obtained. Positive clones producing anti IL-18BP specific antibodies were injected into Balb/C mice pre-primed with pristane for the production of ascites. The isotypes of the antibodies were defined with the use of an anti mouse IgG ELISA (Amersham-Pharmacia Biotech). Mab 582, which was highly reactive with the recombinant and the native IL-18BP (Table 1) was used for the assembly of the IL-18BP specific ELISA (Example 3).

Antibodies were tested by sRIA in the presence of IL-18 (example 5) for their ability to recognize a complex of IL-1 8BPa with IL-18. Most antibodies were unable to recognize

IL-18BP when it was complexed to IL-18. Therefore, these antibodies appear to be directed against the ligand-binding domain of IL-18BPa.

**Example 5 radioimunoassays for the detection of anti IL-18BP antibody producing cell clones.**

Inverted radioimmunoassay (IRIA)·

PVC microtiter plates (Dynatech Laboratories, Alexandria. VA) were coated overnight at 4°C with affinity-purified goat anti-mouse F (ab) 2 antibodies (10 $\mu$g/ml, 100 $\mu$l/well; Jackson ImmunoResearch Labs). The plates were then washed twice with PBS containing 0.05% Tween 20 (washing solution) and blocked with BSA (0.5% in washing solution) for 2 hrs at 37°C. Hybridoma culture supernatants (100 $\mu$l/well) were added and the plates were incubated for 2 hrs at room temperature. The plates were washed three times, $^{125}$I-rIL-18BPa-His6 ($10^5$ cpm in 100 $\mu$l) was added to each well and the plates were incubated for 5 his at 22°C. The plates were then washed three times and individual wells were counted in a gamma counter. Hybridomas generating supernatants, which exhibited bound radioactivity at levers 5 folds higher than the negative control, were considered positive.

Solid phase RIA (sRIA).

rIL-18BPa (5 $\mu$g/ml) was used as the capture antigen and $^{125}$I-goat anti mouse antibodies (100 $\mu$l, $10^5$ cpm) were used for detection. Blocking and washings were done as above. Positive clones were further screened by sRIA for their ability to recognize the IL-18BP isolated from concentrated human urine (Novick et al. 1999). Microtiter plates were coated with ligand-affinity purified urinary IL-18BP (1 $\mu$g/ml), blocked and washed as above. Hybridoma supernatants (100 $\mu$l) were added and detection was done with $^{125}$I-goat anti mouse antibodies ($10^5$ cpm in 100 $\mu$l).

sRIA for antibodies specific for the ligand-binding site of IL-18BP.

Microtiter plates were coated with either urinary IL-18BP (0.5 $\mu$g/ml) or rIL-18BPa (5 $\mu$g/ml), blocked and washed as in sRIA. Recombinant human IL-18 (50$\mu$l) was added to a final concentration of 1.5 $\mu$g/ml (45 min at room temperature). followed by the addition of hybridoma supernatants (50 $\mu$l). Detection was done with $^{125}$I-goal anti mouse antibodies ($10^5$cpm in 100 $\mu$l.2h at room temperature).

Example 6: Myocardial IL-18 content following LPS.

TNF$\alpha$ and 1L-1$\beta$ have been implicated in cardiac dysfunction during sepsis. since IL-18 is a pro-inflammatory cytokine know to mediate the production of TNF$\alpha$ and IL-1$\beta$, the concentration of IL-18 on LPS-induced cardiac dysfunction was tested.

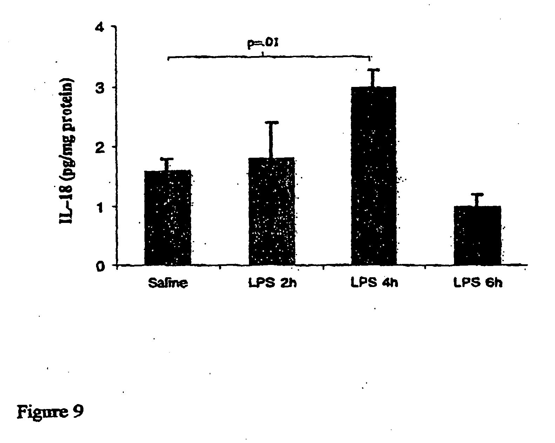

Mice were treated with either vehicle (saline) or LPS. Hearts were harvested at 2, 4 and 6 hours following LPS administration and homogenized to determine myocardial IL-18 content by ELISA (Kit purchased from R&D Systems (Minneapolis MN). The results in Fig. 9 show a two-fold increase in myocardial IL-18 content at 4 hours following LPS administration, which indicates that IL-18 is involved in cardiac dysfunction during sepsis.

**Example 7: Effect of neutralization of IL-18 on LPS-induced myocardial dysfunction.**

In the previous example an increase in the IL-18 content has been found in cardiac dysfunction induced by LPS. Therefore, the effect of neutralization of IL-18 on LPS-induced myocardial dysfunction was evaluated.

Myocardial function was determined by an isovolumetric, nonrecirculating Langendorff technique as described previously (Meng et al. 1998). Isolated hearts were perfused with normothermic Krebs-Henseleit solution containing 11.0 mmol/l glucose, 1.2 mmol/l $CaCl_2$, 4.7 mmol/l KCL, 25 mmol/l $NaHCO_3$, 119 mmol/l NaCl, 1.17 mmol/l $MgSO_4$ and 1.18 mmol/l $KH_2PO_4$. A latex balloon was inserted in the left ventricle via the left atrium and inflated with water to achieve a left ventricular and diastolic pressure (LVEDP) of 10 mmHg. Pacing wires were attached to the right atrium and hearts were paced at 300 beats per minute. Coronary flow was quantified by collecting the effluent from the pulmonary arteries. Myocardial temperature was maintained at 37°C. Left ventricular developed pressure (LVDP), its first derivatives (+dP/dt, -dP/dt) and LVEDP were continuously recorded by a computerized pressure amplifier-digitizer (Maclab 8, AD Instrument, Cupertino, CA). After a 20 minutes equilibration period LVDP and +/-dP/dt were determined at varied LVEDP levels (10, 15, and 20 mmHg).

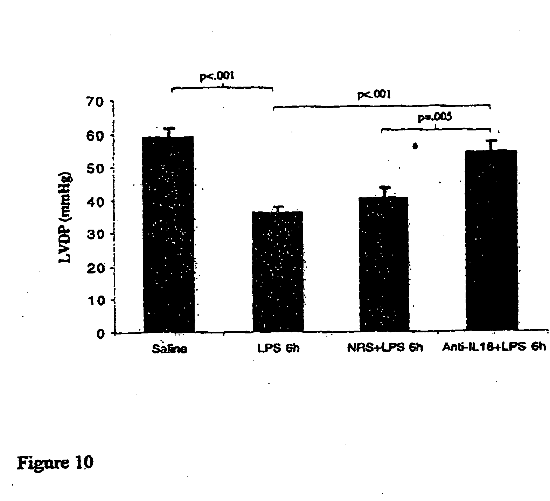

Following LPS administration, the left ventricular developed pressure (LVDP) was reduced by 38% compared to saline controls (36.3+/- 1.9 mmHg vs. 59.1+/- mmHg, P<0.001. Figure 10) Pre treatment of mice 30 minutes prior to LPS administration with normal rabbit serum (NRS) had minimal influence on LPS-induced myocardial dysfunction: however pretreatment with IL-18 neutralizing antibody abrogated dysfunction (Figure 10). Coronary flow was not different between the groups (not shown).

The results indicate that neutralization of IL-18 protects against LPS-induced myocardial dysfunction.

REFERENCES

Aestiz, M.E. and Rackow, E.C. (1998)"Septic shock" THE LANCET 351,1501-5.

Aiura, K., Gelfand, J.A., Burke, J.F., Thompson, R.C. and Dinarello, C.A. (1993) "Interleukin-1 (IL-1) receptor antagonist prevents Staphylococcus epidermidis-induced hypotension and reduces circulating levels of tumor necrosis factor and IL-1 beta in rabbits." Infect Immun 61,3342-50.

Anderson, D.M., Maraskovsky, E., Billingsley, W.L., Dougall, W.C., Tometsko, M.E., Roux, E.R., Teepe, M.C., DuBose, R..F, Cosman, D., Galibert, L. (1997)"A. homologue of the TNF receptor and its ligand enhance T-cell growth and dendritic-cell function." Nature, 390,175-179. Bazan, J. F., Timans, J. C. and Kaselein, R. A. (1996) "A newly defined interleukin-1?"' Nature 379, 591.

Blumberg; R.S. and Strober, W. (2001) "Prospects for research in inflammatory bowel disease" JAMA 283,643-7.

Cleveland, M.G., Gorham, J.D., Murphy, T.L.. Tuomanen, E. and Murphy, K.M.( 1996)" Lipoteichoic acid preparations of Gram-positive bacteria induce interleukin-12 through a CD 14-dependent pathway" Infect Immun 64, 1906-12.

Dinarello CA. (1996) "Biologic basis for interleukin-1 in disease." Blood 87,2095-147. Elliott, M.J., Maini, R.N., Feldmann, M., Long-Fox. A., Charles, P., Bijl. H., and Woody, J.N., (1994)" Repeated therapy with monoclonal antibody to tumour necrosis factor alpha (cA2) in patients with rheumatoid arthritis." Lancet 344, 1125-1127.

Endo, S., Inada, K., Yamada, Y., Wakabayashi, G., Ishikura, H., Tanaka, T. and Sato, S. (2000)" Interleukin 18 (IL-18) levels in patients with sepsis." J Med 31, 15-20. Engelmann, H., Aderka, D., Rubinstein, M., Rotman, D. and Wallach. D. (1989)" A tumor necrosis factor-binding protein purified to homogeneity from human urine protects cells from tumor necrosis factor toxicity" J. Biol. Chem. 264,11974-11980.

Engelmann, H., Novick, D. and Wallach, D. (1990)"Two tumor necrosis factor-binding proteins purified from human urine. Evidence for immunological cross-reactivity with cell surface tumor necrosis factor receptors." J. Biol. Chem. 265,1531-1536.

Fiocchi, C. (1999) "From immune activation to gut tissue injury: the pieces of the puzzle are coming together."Gastroenterology 117,1238-46.
Foss DL, Zilliox MJ, Murtaugh MP.(2001)"Bacterially induced activation of interleukin-18 in porcine intestinal mucosa." Vet Immunol Immunopathol 78,263-77.

Ghayur, T., Banerjee, S., Hugunin, M., Butler, D., Herzog, L., Carter, A., Quintal, L., Sekut, L., Talanian, R.,. Paskind, M., Wong, W., Kamen, R., Tracey, D., and Allen, H. (1997) "Caspase-1 processes IFN-gamma-inducing factor and regulates LPS-induced IFN-gamma production." Nature 386, 619-623.

Grantham, R. (1974)"Amino acid difference formula to help explain protein evolution" Science 185. 862-4.

Grobmyer, S.R., Lin, E., Lowry, S.F., Rivadeneira, D.E., Potter, S., Barie, P.S. and Nathan, C.F. (2000)." Elevation of IL-18 in human sepsis." J Clin Immunol 20, 212-5.

Gu, Y., Kuida, K., Tsutsui, H.. Ku, G., Hsiao, K., Fleming. M. A., Hayashi, N., Higashino, K., Okamura, H., Nakanishi, K., Kurimoto. M., Tanimoto, T., Flavell, R. A., Sato, V., Harding, M. W., Livingston, D. J., and Su, M. S. (1997) "Activation of interferon-gamma inducing factor mediated by interleukin-1beta converting enzyme." Science 275, 206-209.

Gupta, D., Kirkland, T.N., Viriyakosol, S. and Dziarski, R. (1996) "CD14 is a cell-activating receptor for bacterial peptidoglycan." J Biol Chem 271,23310-6.

Hachicha, M., Rathanaswami, P., Naccache, P.H. and McColl, S.R. (1998) "Regulation of chemokine gene expression in human peripheral blood neutrophils phagocytosing microbial pathogens." J Immunol 160, 449-54.

Henderson, B., Poole, S. and Wilson, M. (1996) "Bacterial modulins: a novel class of virulence factors which cause host tissue pathology by inducing cytokine synthesis." Microbiol Rev 60, 316-41.

Karp, C.L., Biron, C.A. and Irani, D.N. (2000) "Interferon $\beta$ in multiple sclerosis: is IL-12 suppression the key?" Immunology today 21, 24-8.

Kim, S.H., Eisenstein, M., Reznikov, L., Fantuzzi, G., Novick, D., Rubinstein, M. and Dinarello, C.A.(2000)" Structural requirements of six naturally occurring isoforms of the IL-18 binding protein to inhibit IL-18."Proc Natl Acad Sci U S A 97, 1190-5.

Kim, S.H., Reznikov, L.L., Stuyt, R.J., Selzman, C.H., Fantuzzi, G., Hoshino, T., Young, H.A. and Dinarello, C.A. (2001)" Functional reconstitution and regulation of IL-18 activity by the IL-18R beta chain." J Immunol 166,148-54.

Knaus, W.A., Harrell, F.E., Fisher, C.J. Jr, Wagner, D.P.; Opal, SM., Sadoff, J.C., Draper, E.A., Walawander, C.A., Conboy, K. and Grasela, T.H.(1993) "The clinical evaluation of new drugs for sepsis. A prospective study design based on survival analysis." JAMA 1993 270, 1233-41.

Knight, D.M., Trinh, H.. Le, J., Siegel, S.. Shealy, D., McDonough. M., Scallon, B.. Moore, M.A., Vilcek and J., Daddona, P., (1993) "Construction and initial characterization of a mouse-human chimeric anti-TNF antibody.Mol Immunol" 16, 1443-53.

Kohno, K., J. Kataoka. T. Ohtsuki, Y. Suemoto, I. Okamoto, M. Usui, M. Ikeda, and M. Kurimoto. (1997) "IFN-gamma-inducing factor (IGIF) is a costimulatory factor on the activation of Th1 but not Th2 cells and exerts its effect independently of IL-12." J. Immunol. 158:1541-1550.

Liu, B., Novick, D., Kim, S.H. and Rubinstein, M. (2000) "Production of a biologically active human interleukin 18 requires its prior synthesis as pro-IL-18 cytokine." 12, 1519-1525.

Maliszewski, C.R., Sato, T.A., Vanden Bos, T., Waugh, S., Dower, S.K., Slack, J., Beckmann, M.P. and Grabstein KH. (1990) "Cytokine receptors and B cell functions. I. Recombinant soluble receptors specifically inhibit IL- 1- and IL-4-induced B cell activities in vitro." J Immunol 144, 3028-33.

Mattner, F., Fischer, S., Guckes, S., Jin, S., Kaulen, H., Schmitt, E., Rude, E. and Germann, T. (1993)" The interleukin-12 subunit p40 specifically inhibits effects of the interleukin-12 heterodimer." Eur J Immunol 23, 2202-8.

Muhl, H., Kampfer, H., Bosmann, M., Frank, S., Radeke, H. and Pfeilschifter J. (2000) " Interferon-gamma mediates gene expression of IL-18 binding protein in nonleukocytic cells." Biochem Biophys Res Commun Jan 267, 960-3.

Nakamura, K., Okamura, H., Wada, M., Nagata, K. and Tamura, T. (1989). "Endotoxin-induced serum factor that stimulates gamma interferon production." Infect-Immun 57,590-5 issn: 0019-9567.

Nakamura. K.. Okamura. H., Nagata, K., Komatsu. T. and Tamura. T. (1993) "Purification of a factor which provides a costimulatory signal for gamma interferon production." Infect. Immun. 61, 64-70.

Nakamura, S.. Otani. T., Ijiri. Y.. otoda, R.. Kurimoto. M. and Orita. K.(2000)" IFN-$\gamma$-dependent and -independent mechanisms in adverse effects caused by concomitant administration of IL-18 and IL-12. The journal of Immunology 164. 3330-6.

Nakanishi K, Yoshimoto T, Tsutsui H, Okamura H. (2001) "Interleukin-18 is a unique cytokine that stimulates both Th1 and Th2 responses depending on its cytokine milieu." Cytokine Growth Factor Rev 12, 53-72.

Nathens AB, Marshall JC. (1996) " Sepsis, SIRS, and MODS: what's in a name?" World J Surg 20,386-91

Netea, M. G., Fantuzzi, G., Kullberg, B.J., Stuyt, R. J. L., Pulido. E. J., McIntyre, Jr. R. C., Joosten, L. A. B., Van der Meer, J.W. M. and Dinarello, C.A.(2000)" Neutralization of IL-18 reduces neutophil tissue accumulation and protects mice against lethal Escherichia coli and Salmonella typhimurium endotoxemia" The journal of immunology 2644-2649.

Novick, D., Engelmann, H., Wallach, D. and Rubinstein. M. (1989) "soluble cytokine receptors are present in normal human urine." J. Exp. Med. 170,1409-14.

Novick, D., Cohen, B. and Rubinstein, M. (1992) "Soluble Interferon-alpha Receptor Molecules Are Present in Body Fluids." FEBS Lett 314,445-8.

Novick, D., Cohen, B. and Rubinstein, M. (1994) "The Human Interferon alpha/beta Receptor - Characterization and Molecular Cloning." Cell 77,391-400.

Novick, D., Kim,S., Fantuzzi, G., Reznikov, L.L., Dinarello, C.A. and Rubinstein, M. (1999) "Interleukin-18 Binding Protein: A Novel Modulator of the Th1 Cytokine Response. Immunity 10, 127,36.

Okamura, H., Tsutsui, H., Komatsu, T., Yutsudo, M., Hakura, A., Tanimoto, T., Torigoe, K., Okura, T., Nukada, Y., Hattori, K., Akita, K., Namba, M., Tanabe, F., Konishi, K., Fukuda, S., and Kurimoto, M. (1995) "Cloning of a new cytokine that induces IFN-gamma production by T cells." Nature 378, 88-91.

Okamura. H., Kashiwamura S., Tsutsui. H.. Yoshimoto. T. and Nakanishi, K. (1998) "Regulation of IFN-γ production by IL-12 and IL-18" Current Opinion in Immunology. 10, 259-264.

Okusawa. S., Gelfand. J.A.. Ikejima. T.. Connolly. R.J. and Dinarello. C.A.(1988) "Interleukin 1 induces a shock-like state in rabbits. Synergism with tumor necrosis factor and the effect of cyclooxygenase inhibition."J Clin Invest 81, 1162-72.

Pomerantz, B.J., Reznikov, L.L.. Harken. A.H. and Dinarello CA.(2001)" Inhibition of caspase 1 reduces human myocardial ischemic dysfunction via inhibition of IL-18 and IL-1 beta." Proc Natl Acad Sci U S A 98, 2871-6.

Puren, A.J., Fantuzzi, G., Dinarello, C.A. (1999) "Gene expression, synthesis, and secretion of interleukin 18 and interleukin 1beta are differentially regulated in human blood mononuclear cells and mouse spleen cells." Proc Natl Acad Sci U S A ,96,2256-61. Sakao, Y., Takeda, K., Tsutsui, H., Kaisho, T., Nomura, F., Okamura, H., Nakanishi, K. and Akira S. (1999) "IL-18-deficient mice are resistant to endotoxin-induced liver injury but highly susceptible to endotoxin shock." Int Immunol 3, 471-80.

Seki, .S, Habu, Y., Kawamura, T., Takeda, K., Dobashi, H.. Ohkawa, T., Hiraide, H. (2000) " The liver as a crucial organ in the first line of host defense: the roles of Kupffer cells, natural killer (NK) cells and NK1.1 Ag+ T cells in T helper 1 immune responses." Immunol Rev 174,35-46.

Simonet, W.S., Lacey, D.L., Dunstan, C.R., Kelley, M., Chang, M.S., Luthy, R., Nguyen, H.Q., Wooden, S., Bennett, L., Boone, T., Shimamoto, G., DeRose, M., Elliott, R., Colombero, A., Tan, H.L., Trail, G., Sullivan, J., Davy, E., Bucay, N., Renshaw-Gegg, L., Hughes, T.M., Hill, D., Pattison, W., Campbell, P., Boyle, W.J. (1997)." Osteoprotegerin: a novel secreted protein involved in the regulation of bone density ". Cell, 89, 309-19.

Skeen, M.J. and Ziegler, H.K.(1995)"Activation of gamma delta T cells for production of IFN-gamma is mediated by bacteria via macrophage-derived cytokines IL-1 and IL-12." J Immunol. 154, 5832-41.

Trinchieri, G. (1998) "Interleukin-12: a cytokine at the interface of inflammation and immunity." Adv Immunol 70,83-243.

Tripp, C.S., Wolf, S.F. and Unanue, E.R. (1993) " Interleukin 12 and tumor necrosis factor alpha are costimulators of interferon gamma production by natural killer cells in severe combined immunodeficiency mice with listeriosis and interleukin 10 is a physiologic antagonist." Proc Natl Acad Sci U S A 90. 3725-9.

Tsutsui, H., K. Nakanishi. K. Matsui. K. Higashino, H. Okamura. Y. Miyazawa, and K. Kaneda. (1996) "IFN-gamma-inducing factor up-regulates Fas ligand-mediated cytotoxic activity of murine natural killer cell clones" . J. Immuno/. 157,3967-73 issn: 0022-1767.

Urushihara, N., Iwagaki, H., Yagi, T., Kohka. H.. Kobashi, K., Morimoto. Y., Yoshino, T.. Tanimoto, T., Kurimoto, M., Tanaka. N. (2000) "Elevation of serum interleukin-18 levels and activation of Kupffer cells in biliary atresia." J Pediatr Surg 35.446-9.

Ushio, S., Namba, M., Okura, T., Hattori, K.. Nukada, Y., Akita, K., Tanabe, F., Konishi, K., Micallef, M., Fujii, M., Torigoe, K., Tanimoto. T., Fukuda, S., Ikeda, M., Okamura, H., and Kurimoto, M. (1996)" Cloning of the cDNA for human IFN-gamma-inducing factor, expression in Escherichia coli, and studies on the biologic activities of the protein." J. Immunol. 156,4274-9.

Vigers, G.P., Anderson, L.J., Caffes, P., Brandhuber, B.J. (1997) "Crystal structure of the type-I interleukin-1 receptor complexed with interleukin-1beta." Nature 386,190-4.

Xiang, Y. and Moss, B. (1999)"Identification of human and mouse homologs of the MC5 1L-53L-54L family of secreted glycoproteins encoded by the Molluseum contagiosum poxvirus." Virology 257, 297-302.

Yasuda, H., Shima, N., Nakagawa; N., Mochizuki, S.I., Yano, K., Fujise, N., Sato, Y., Goto, M., Yamaguchi, K., Kuriyama, M., Kanno, T., Murakami, A., Tsuda, E., Morinaga, T., Higashio, K. (1998) " Identity of osteoclastogenesis inhibitory factor (OCIF) and osteoprotegerin (OPG): a mechanism by which OPG/OCIF inhibits osteoclastogenesis in vitro." Endocrinology, 139, 1329-37.

**Claims**

1. The use of an inhibitor of IL-18 in the manufacture of a medicament for the treatment and/or prevention of sepsis related cardiac dysfunction, wherein the inhibitor is selected from antibodies against IL-18 and the IL-18 binding proteins IL-18 BPa and IL-18 BPc.

2. The use according to claim 1, wherein the IL-18 binding protein is PEG-conjugated.

3. The use according to claim 1, wherein the inhibitor of IL-18 is an anti IL-18 specific antibody selected from chimeric, humanized and human antibodies.

4. The use according to any one of the preceding claims, wherein the medicament further comprises an inhibitor of a cytokine, wherein the cytokine is selected from the tumor necrosis factor $\alpha$ (TNF-$\alpha$) and IL-1$\beta$, and wherein said TNF-$\alpha$ inhibitor is the soluble portion of TNFRI or TNFRII and the IL-1$\beta$ inhibitor is the IL-1 receptor antagonist.

5. The use of an expression vector comprising the coding sequence of an inhibitor of IL-18 selected from antibodies against IL-18, and the IL-18 binding proteins IL-18 BPa and IL-18 BPc in the manufacture of a medicament for the treatment and/or prevention of sepsis related cardiac dysfunction.

6. The use of an expression vector, which is defined as in claim 5, for the manufacture of a medicament for the treatment and/or prevention of sepsis related cardiac dysfunction by gene therapy.

7. The use of a cell that has been genetically modified to produce an inhibitor of IL-18 selected from antibodies against IL-18 and the IL-18 binding proteins IL-18 BPa and IL-18 BPc in the manufacture of a medicament for the treatment and/or prevention of sepsis related cardiac dysfunction.

**Patentansprüche**

1. Verwendung eines IL-18 Inhibitors bei der Herstellung eines Medikaments zur Behandlung und/oder Prävention von Sepsis bezogener Herzfunktionsstörung, wobei der Inhibitor ausgewählt ist aus Antikörpern gegen IL-18 und die IL-18 Bindeproteine IL-18 BPa und IL-18 BPc.

2. Verwendung nach Anspruch 1, wobei das IL-18 Bindeprotein mit PEG konjugiert ist.

3. Verwendung nach Anspruch 1, wobei der IL-18 Inhibitor ein anti-IL-18 spezifischer Antikörper ist, ausgewählt aus chimeren, humanisierten und humanen Antikörpern.

4. Verwendung nach einem der vorherigen Ansprüche, wobei das Medikament weiter einen Inhibitor eines Cytokins umfasst, wobei das Cytokin ausgewählt ist aus dem Tumornekrosefaktor $\alpha$ (TNF-$\alpha$) und IL-1$\beta$, und wobei der TNF-$\alpha$ Inhibitor der lösliche Teil von TNFRI und TNFRII ist und der IL-1$\beta$ Inhibitor der IL-1 Rezeptor-Antagonist ist.

5. Verwendung eines Expressionsvektors, der die Kodierungssequenz eines IL-18 Inhibitors, ausgewählt aus Antikörpern gegen IL-18 und die IL-18 Bindeproteine IL-18 BPa und IL-18 BPc, umfasst, bei der Herstellung eines Medikaments zur Behandlung und/oder Prävention von Sepsis bezogener Herzfunktionsstörung.

6. Verwendung eines Expressionsvektors, der wie in Anspruch 5 definiert ist, zur Herstellung eines Medikaments zur Behandlung und/oder Prävention von Sepsis bezogener Herzfunktionsstörung durch Gentherapie.

7. Verwendung einer Zelle, die genetisch modifiziert wurde, um einen IL-18 Inhibitor, ausgewählt aus Antikörpern gegen IL-18 und die IL-18 Bindeproteine IL-18 BPa und IL-18 BPc, zu erzeugen, bei der Herstellung eines Medikaments zur Behandlung und/oder Prävention von Sepsis bezogener Herzfunktionsstörung.

**Revendications**

1. Utilisation d'un inhibiteur de l'IL-18 dans la fabrication d'un médicament destiné au traitement et/ou à la prévention de l'insuffisance cardiaque liée à une septicémie, l'inhibiteur étant choisi parmi les anticorps anti-IL-18 et les protéines de liaison l'IL-18 IL-18 BPa et IL-18BPc.

2. Utilisation selon la revendication 1, dans laquelle la protéine de liaison à l'IL-18 est conjuguée au PEG.

3. Utilisation selon la revendication 1, dans laquelle l'inhibiteur de l'IL-18 est un anticorps anti-IL-18 spécifique choisi parmi des anticorps chimériques, humanisés et humains.

4. Utilisation selon l'une quelconque des revendications précédentes, dans laquelle le médicament comprend en outre un inhibiteur d'une cytokine, la cytokine étant choisie parmi le facteur onconécrosant $\alpha$ (TNF-$\alpha$) et l'IL-1$\beta$, et ledit inhibiteur du TNF-$\alpha$ étant la partie soluble du TNFRI ou du TNFRII et l'inhibiteur de l'IL-1$\beta$ étant l'antagoniste du récepteur de l'IL-1.

5. Utilisation d'un vecteur d'expression comprenant la séquence codante d'un inhibiteur de l'IL-18 choisi parmi les anticorps anti-IL-18, et les protéines de liaison à l'IL-18 IL-18 BPa et IL-18 BPc dans la fabrication d'un médicament destiné au traitement et/ou à la prévention de l'insuffisance cardiaque liée à une septicémie.

6. Utilisation d'un vecteur d'expression, qui est défini comme dans la revendication 5, pour la fabrication d'un médicament destiné au traitement et/ou à la prévention de l'insuffisance cardiaque liée à une septicémie par thérapie génique.

7. Utilisation d'une cellule qui a été génétiquement modifiée pour produire un inhibiteur de l'IL-18 choisi parmi les anticorps anti-IL-18 et les protéines de liaison à l'IL-18 IL-18 BPa et IL-18 BPc dans la fabrication d'un médicament destiné au traitement et/ou à la prévention de l'insuffisance cardiaque liée à une septicémie.

**Figure 1**

Figure 2 A

**Figure 2 B**

**Figure 3**

**Figure 4**

**Figure 5**

Figure 6

**Figure 7**

**Figure 8**

Figure 9

**Figure 10**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 29146301 P
- WO 9213095 A
- EP 0974600 A
- WO 0076310 A

- WO 9953049 A
- US 6162963 A
- AU 5336100
- WO 9109955 A

**Non-patent literature cited in the description**

- **Aestiz, M.E. ; Rackow, E.C.** Septic shock. *THE LANCET,* 1998, vol. 351, 1501-5
- **Aiura, K. ; Gelfand, J.A. ; Burke, J.F. ; Thompson, R.C. ; Dinarello, C.A.** Interleukin-1 (IL-1) receptor antagonist prevents Staphylococcus epidermidis-induced hypotension and reduces circulating levels of tumor necrosis factor and IL-1 beta in rabbits. *Infect Immun,* 1993, vol. 61, 3342-50
- **Anderson, D.M. ; Maraskovsky, E. ; Billingsley, W.L. ; Dougall, W.C. ; Tometsko, M.E. ; Roux, E.R. ; Teepe, M.C. ; DuBose, R..F ; Cosman, D. ; Galibert, L.** A. homologue of the TNF receptor and its ligand enhance T-cell growth and dendritic-cell function. *Nature,* 1997, vol. 390, 175-179
- **Bazan, J. F. ; Timans, J. C. ; Kaselein, R. A.** A newly defined interleukin-1?. *Nature,* 1996, vol. 379, 591
- **Blumberg; R.S. ; Strober, W.** Prospects for research in inflammatory bowel disease. *JAMA,* 2001, vol. 283, 643-7
- **Cleveland, M.G. ; Gorham, J.D. ; Murphy, T.L. ; Tuomanen, E. ; Murphy, K.M.** Lipoteichoic acid preparations of Gram-positive bacteria induce interleukin-12 through a CD 14-dependent pathway. *Infect Immun,* 1996, vol. 64, 1906-12
- **Dinarello CA.** Biologic basis for interleukin-1 in disease. *Blood,* 1996, vol. 87, 2095-147
- **Elliott, M.J. ; Maini, R.N. ; Feldmann, M. ; Long-Fox. A. ; Charles, P. ; Bijl. H. ; Woody, J.N.** Repeated therapy with monoclonal antibody to tumour necrosis factor alpha (cA2) in patients with rheumatoid arthritis. *Lancet,* 1994, vol. 344, 1125-1127
- **Endo, S. ; Inada, K. ; Yamada, Y. ; Wakabayashi, G. ; Ishikura, H. ; Tanaka, T. ; Sato, S.** Interleukin 18 (IL-18) levels in patients with sepsis. *J Med,* 2000, vol. 31, 15-20

- **Engelmann, H. ; Aderka, D. ; Rubinstein, M. ; Rotman, D. ; Wallach. D.** A tumor necrosis factor-binding protein purified to homogeneity from human urine protects cells from tumor necrosis factor toxicity. *J. Biol. Chem.,* 1989, vol. 264, 11974-11980
- **Engelmann, H. ; Novick, D. ; Wallach, D.** Two tumor necrosis factor-binding proteins purified from human urine. Evidence for immunological cross-reactivity with cell surface tumor necrosis factor receptors. *J. Biol. Chem.,* 1990, vol. 265, 1531-1536
- **Fiocchi, C.** From immune activation to gut tissue injury: the pieces of the puzzle are coming together. *Gastroenterology,* 1999, vol. 117, 1238-46
- **Foss DL ; Zilliox MJ ; Murtaugh MP.** Bacterially induced activation of interleukin-18 in porcine intestinal mucosa. *Vet Immunol Immunopathol,* 2001, vol. 78, 263-77
- **Ghayur, T. ; Banerjee, S. ; Hugunin, M. ; Butler, D. ; Herzog, L. ; Carter, A. ; Quintal, L. ; Sekut, L. ; Talanian, R. ; Paskind, M.** Caspase-1 processes IFN-gamma-inducing factor and regulates LPS-induced IFN-gamma production. *Nature,* 1997, vol. 386, 619-623
- **Grantham, R.** Amino acid difference formula to help explain protein evolution. *Science,* 1974, vol. 185, 862-4
- **Grobmyer, S.R. ; Lin, E. ; Lowry, S.F. ; Rivadeneira, D.E. ; Potter, S. ; Barie, P.S. ; Nathan, C.F.** Elevation of IL-18 in human sepsis. *J Clin Immunol,* 2000, vol. 20, 212-5
- **Gu, Y. ; Kuida, K. ; Tsutsui, H. ; Ku, G. ; Hsiao, K. ; Fleming. M. A. ; Hayashi, N. ; Higashino, K. ; Okamura, H. ; Nakanishi, K.** Activation of interferon-gamma inducing factor mediated by interleukin-1beta converting enzyme. *Science,* 1997, vol. 275, 206-209
- **Gupta, D. ; Kirkland, T.N. ; Viriyakosol, S. ; Dziarski, R.** CD14 is a cell-activating receptor for bacterial peptidoglycan. *J Biol Chem,* 1996, vol. 271, 23310-6

- **Hachicha, M. ; Rathanaswami, P. ; Naccache, P.H. ; McColl, S.R.** Regulation of chemokine gene expression in human peripheral blood neutrophils phagocytosing microbial pathogens. *J Immunol,* 1998, vol. 160, 449-54
- **Henderson, B. ; Poole, S. ; Wilson, M.** Bacterial modulins: a novel class of virulence factors which cause host tissue pathology by inducing cytokine synthesis. *Microbiol Rev,* 1996, vol. 60, 316-41
- **Karp, C.L. ; Biron, C.A. ; Irani, D.N.** Interferon β in multiple sclerosis: is IL-12 suppression the key?. *Immunology today,* 2000, vol. 21, 24-8
- **Kim, S.H. ; Eisenstein, M. ; Reznikov, L. ; Fantuzzi, G. ; Novick, D. ; Rubinstein, M. ; Dinarello, C.A.** Structural requirements of six naturally occurring isoforms of the IL-18 binding protein to inhibit IL-18. *Proc Natl Acad Sci U S A,* 2000, vol. 97, 1190-5
- **Kim, S.H. ; Reznikov, L.L. ; Stuyt, R.J. ; Selzman, C.H. ; Fantuzzi, G. ; Hoshino, T. ; Young, H.A. ; Dinarello, C.A.** Functional reconstitution and regulation of IL-18 activity by the IL-18R beta chain. *J Immunol,* 2001, vol. 166, 148-54
- **Knaus, W.A. ; Harrell, F.E. ; Fisher, C.J. Jr ; Wagner, D.P. ; Opal, SM. ; Sadoff, J.C. ; Draper, E.A. ; Walawander, C.A. ; Conboy, K. ; Grasela, T.H.** The clinical evaluation of new drugs for sepsis. A prospective study design based on survival analysis. *JAMA,* 1993, vol. 270, 1233-41
- **Knight, D.M. ; Trinh, H. ; Le, J. ; Siegel, S. ; Shealy, D. ; McDonough. M. ; Scallon, B. ; Moore, M.A. ; Vilcek and J. ; Daddona, P.** Construction and initial characterization of a mouse-human chimeric anti-TNF antibody. *Mol Immunol,* 1993, vol. 16, 1443-53
- **Kohno, K. ; J. Kataoka ; T. Ohtsuki ; Y. Suemoto ; I. Okamoto ; M. Usui ; M. Ikeda ; M. Kurimoto.** IFN-gamma-inducing factor (IGIF) is a costimulatory factor on the activation of Th1 but not Th2 cells and exerts its effect independently of IL-12. *J. Immunol.,* 1997, vol. 158, 1541-1550
- **Liu, B. ; Novick, D. ; Kim, S.H. ; Rubinstein, M.** *Production of a biologically active human interleukin 18 requires its prior synthesis as pro-IL-18 cytokine.,* 2000, vol. 12, 1519-1525
- **Maliszewski, C.R. ; Sato, T.A. ; Vanden Bos, T. ; Waugh, S. ; Dower, S.K. ; Slack, J. ; Beckmann, M.P. ; Grabstein KH.** Cytokine receptors and B cell functions. I. Recombinant soluble receptors specifically inhibit IL-1- and IL-4-induced B cell activities in vitro. *J Immunol,* 1990, vol. 144, 3028-33
- **Mattner, F. ; Fischer, S. ; Guckes, S. ; Jin, S. ; Kaulen, H. ; Schmitt, E. ; Rude, E. ; Germann, T.** The interleukin- 12 subunit p40 specifically inhibits effects of the interleukin-12 heterodimer. *Eur J Immunol,* 1993, vol. 23, 2202-8
- **Muhl, H. ; Kampfer, H. ; Bosmann, M. ; Frank, S. ; Radeke, H. ; Pfeilschifter J.** Interferon-gamma mediates gene expression of IL-18 binding protein in nonleukocytic cells. *Biochem Biophys Res Commun Jan,* 2000, vol. 267, 960-3
- **Nakamura, K. ; Okamura, H. ; Wada, M. ; Nagata, K. ; Tamura, T.** Endotoxin-induced serum factor that stimulates gamma interferon production. *Infect-Immun,* 1989, vol. 57, ISSN 0019-9567, 590-5
- **Nakamura. K. ; Okamura. H. ; Nagata, K. ; Komatsu. T. ; Tamura. T.** Purification of a factor which provides a costimulatory signal for gamma interferon production. *Infect. Immun.,* 1993, vol. 61, 64-70
- **Nakamura, S. ; Otani. T. ; Ijiri. Y. ; otoda, R. ; Kurimoto. M. ; Orita. K.** IFN-γ-dependent and -independent mechanisms in adverse effects caused by concomitant administration of IL-18 and IL-12. *The journal of Immunology,* 2000, vol. 164, 3330-6
- **Nakanishi K ; Yoshimoto T ; Tsutsui H ; Okamura H.** Interleukin-18 is a unique cytokine that stimulates both Th1 and Th2 responses depending on its cytokine milieu. *Cytokine Growth Factor Rev,* 2001, vol. 12, 53-72
- **Nathens AB ; Marshall JC.** Sepsis, SIRS, and MODS: what's in a name?. *World J Surg,* 1996, vol. 20, 386-91
- **Netea, M. G. ; Fantuzzi, G. ; Kullberg, B.J. ; Stuyt, R. J. L. ; Pulido. E. J. ; McIntyre, Jr. R. C. ; Joosten, L. A. B. ; Van der Meer, J.W. M. ; Dinarello, C.A.** Neutralization of IL-18 reduces neutophil tissue accumulation and protects mice against lethal Escherichia coli and Salmonella typhimurium endotoxemia. *The journal of immunology,* 2000, 2644-2649
- **Novick, D. ; Engelmann, H. ; Wallach, D. ; Rubinstein. M.** soluble cytokine receptors are present in normal human urine. *J. Exp. Med.,* 1989, vol. 170, 1409-14
- **Novick, D. ; Cohen, B. ; Rubinstein, M.** Soluble Interferon-alpha Receptor Molecules Are Present in Body Fluids. *FEBS Lett,* 1992, vol. 314, 445-8
- **Novick, D. ; Cohen, B. ; Rubinstein, M.** The Human Interferon alpha/beta Receptor - Characterization and Molecular Cloning. *Cell,* 1994, vol. 77, 391-400
- **Novick, D. ; Kim,S. ; Fantuzzi, G. ; Reznikov, L.L. ; Dinarello, C.A. ; Rubinstein, M.** Interleukin-18 Binding Protein: A Novel Modulator of the Th1 Cytokine Response. *Immunity,* 1999, vol. 10 (36), 127
- **Okamura, H. ; Tsutsui, H. ; Komatsu, T. ; Yutsudo, M. ; Hakura, A. ; Tanimoto, T. ; Torigoe, K. ; Okura, T. ; Nukada, Y. ; Hattori, K.** Cloning of a new cytokine that induces IFN-gamma production by T cells. *Nature,* 1995, vol. 378, 88-91

- **Okamura. H. ; Kashiwamura S. ; Tsutsui. H. ; Yoshimoto. T. ; Nakanishi, K.** Regulation of IFN-γ production by IL-12 and IL-18. *Current Opinion in Immunology,* 1998, vol. 10, 259-264
- **Okusawa. S. ; Gelfand. J.A. ; Ikejima. T. ; Connolly. R.J. ; Dinarello. C.A.** Interleukin 1 induces a shock-like state in rabbits. Synergism with tumor necrosis factor and the effect of cyclooxygenase inhibition. *J Clin Invest,* 1988, vol. 81, 1162-72
- **Pomerantz, B.J. ; Reznikov, L.L. ; Harken. A.H. ; Dinarello CA.** Inhibition of caspase 1 reduces human myocardial ischemic dysfunction via inhibition of IL-18 and IL-1 beta. *Proc Natl Acad Sci U S A,* 2001, vol. 98, 2871-6
- **Puren, A.J. ; Fantuzzi, G. ; Dinarello, C.A.** Gene expression, synthesis, and secretion of interleukin 18 and interleukin 1beta are differentially regulated in human blood mononuclear cells and mouse spleen cells. *Proc Natl Acad Sci U S A,* 1999, vol. 96, 2256-61
- **Sakao, Y. ; Takeda, K. ; Tsutsui, H. ; Kaisho, T. ; Nomura, F. ; Okamura, H. ; Nakanishi, K. ; Akira S.** IL-18-deficient mice are resistant to endotoxin-induced liver injury but highly susceptible to endotoxin shock. *Int Immunol,* 1999, vol. 3, 471-80
- **Seki, .S ; Habu, Y. ; Kawamura, T. ; Takeda, K. ; Dobashi, H. ; Ohkawa, T. ; Hiraide, H.** The liver as a crucial organ in the first line of host defense: the roles of Kupffer cells, natural killer (NK) cells and NK1.1 Ag+ T cells in T helper 1 immune responses. *Immunol Rev,* 2000, vol. 174, 35-46
- **Simonet, W.S. ; Lacey, D.L. ; Dunstan, C.R. ; Kelley, M. ; Chang, M.S. ; Luthy, R. ; Nguyen, H.Q. ; Wooden, S. ; Bennett, L. ; Boone, T.** Osteoprotegerin: a novel secreted protein involved in the regulation of bone density. *Cell,* 1997, vol. 89, 309-19
- **Skeen, M.J. ; Ziegler, H.K.** Activation of gamma delta T cells for production of IFN-gamma is mediated by bacteria via macrophage-derived cytokines IL-1 and IL-12. *J Immunol.,* 1995, vol. 154, 5832-41
- **Trinchieri, G.** Interleukin-12: a cytokine at the interface of inflammation and immunity. *Adv Immunol,* 1998, vol. 70, 83-243
- **Tripp, C.S. ; Wolf, S.F. ; Unanue, E.R.** Interleukin 12 and tumor necrosis factor alpha are costimulators of interferon gamma production by natural killer cells in severe combined immunodeficiency mice with listeriosis and interleukin 10 is a physiologic antagonist. *Proc Natl Acad Sci U S A,* 1993, vol. 90, 3725-9
- **Tsutsui, H. ; K. Nakanishi. ; K. Matsui ; K. Higashino ; H. Okamura ; Y. Miyazawa ; K. Kaneda.** IFN-gamma-inducing factor up-regulates Fas ligand-mediated cytotoxic activity of murine natural killer cell clones. *J. Immuno/.,* 1996, vol. 157, ISSN 0022-1767, 3967-73
- **Urushihara, N. ; Iwagaki, H. ; Yagi, T. ; Kohka. H. ; Kobashi, K. ; Morimoto. Y. ; Yoshino, T. ; Tanimoto, T. ; Kurimoto, M. ; Tanaka. N.** Elevation of serum interleukin-18 levels and activation of Kupffer cells in biliary atresia. *J Pediatr Surg,* 2000, vol. 35, 446-9
- **Ushio, S. ; Namba, M. ; Okura, T. ; Hattori, K. ; Nukada, Y. ; Akita, K. ; Tanabe, F. ; Konishi, K. ; Micallef, M. ; Fujii, M.** Cloning of the cDNA for human IFN-gamma-inducing factor, expression in Escherichia coli, and studies on the biologic activities of the protein. *J. Immunol.,* 1996, vol. 156, 4274-9
- **Vigers, G.P. ; Anderson, L.J. ; Caffes, P. ; Brandhuber, B.J.** Crystal structure of the type-I interleukin-1 receptor complexed with interleukin-1beta. *Nature,* 1997, vol. 386, 190-4
- **Xiang, Y. ; Moss, B.** Identification of human and mouse homologs of the MC5 1L-53L-54L family of secreted glycoproteins encoded by the Molluseum contagiosum poxvirus. *Virology,* 1999, vol. 257, 297-302
- **Yasuda, H. ; Shima, N. ; Nakagawa; N. ; Mochizuki, S.I. ; Yano, K. ; Fujise, N. ; Sato, Y. ; Goto, M. ; Yamaguchi, K. ; Kuriyama, M.** Identity of osteoclastogenesis inhibitory factor (OCIF) and osteoprotegerin (OPG): a mechanism by which OPG/OCIF inhibits osteoclastogenesis in vitro. *Endocrinology,* 1998, vol. 139, 1329-37